# EUROPEAN PATENT APPLICATION

(11) **EP 2 269 630 A1**
(43) Date of publication of application: **05.01.2011**
(21) Application number: 09007841.1
(22) Date of filing: 15.06.2009
(51) Int. Cl.: A61K 38/45, A61K 31/575, A61K 39/395

(54) **Novel anti-nematode therapies targetting STRM-1**

(71) Applicant: Max-Planck-Gesellschaft zur Förderung der Wissenschaften e.V., 80539 München (DE); Technische Universität Dresden, 01062 Dresden (DE)
(72) Inventor: Kurzchalia, Teymuras, 10435 Berlin (DE); Hannich, Thomas, 74240 Gaillard (FR); Entchev, Eugeni, 01309 Dresden (DE); Knölker, Hans-Joachim, 01187 Dresden (DE); Martin, René, 01159 Dresden (DE)
(74) Representative: Vossius & Partner

(57) **Abstract**

The present invention relates to a compound for use as an anti-helminthic agent, said compound being selected from (i) a compound interfering with the transcription or translation of a nucleic acid sequence encoding a polypeptide having a function of introducing a methyl group at a specific position into the sterol nucleus of a sterol which nucleic acid sequence is (a) a nucleic acid sequence encoding a polypeptide comprising or consisting of the amino acid sequence of SEQ ID NO: 1; (b) a nucleic acid sequence comprising or consisting of the sequence of SEQ ID NO: 2; (c) a nucleic acid sequence which encodes a polypeptide which is at least 70% identical to the sequence of SEQ ID NO: 1;(d) the nucleic acid sequence of (b) wherein T is U; (e) a nucleic acid sequence hybridizing to the complementary strand of a nucleic acid molecule of any of (a) to (d); or (f) a nucleic acid sequence being degenerate to the sequence of the nucleic acid molecule of (d) or (e), wherein said compound is an antisense molecule, siRNA, shRNA, miRNA, aptamer, antibody or a ribozyme, and (ii) a compound interfering with the function of the polypeptide encoded by the nucleic acid sequence of (i) said compound being an antibody, enzyme, peptide or a small organic molecule.

SEQUENCE LISTING

<110> Max-Planck-Gesellschaft zur Förderung der Wissenschaften
e.V.
Technische Universität Dresden

<120> A nucleic acid molecule encoding a polypeptide which triggers
sterol-ring methylation

<130> R1859 EP

<160> 2

<170> PatentIn version 3.3

<210> 1
<211> 334
<212> PRT
<213> Caenorhabditis elegans

<400> 1

Met Ser Ile Asn Met Asn Ala Asn Phe Leu Lys Leu Leu Thr His Phe
1               5                   10                  15

Arg Arg His Asp Leu Thr Asn Phe Lys Ser Glu His Asp Thr Leu Tyr
            20                  25                  30

Glu Lys Ala Leu Glu Thr Gly Asp His Leu Glu Val Thr Ser His Tyr
        35                  40                  45

Tyr Ser Val Met Ser Thr Val Ile Asp Glu Tyr Phe Gly Gly Asn Phe
    50                  55                  60

His Phe Val Pro Pro Lys Phe Glu Gly Gln Lys Leu Glu Glu Ala Leu
65                  70                  75                  80

Lys Ser Leu His Cys His Ile Ala Glu Lys Leu Glu Leu Ser Glu Asn
                85                  90                  95

Val His Cys Leu Asp Ile Gly Cys Gly Ile Gly Gly Val Met Leu Asp
            100                 105                 110

Ile Ala Asp Phe Gly Ala Lys Leu Thr Gly Val Thr Ile Ala Pro Asn
        115                 120                 125

Glu Ala Glu Ile Gly Asn Glu Lys Phe Ala Asn Met Gly Ile Ser Asp
    130                 135                 140

Arg Cys Lys Ile Val Ala Ala Asp Cys Gln Lys Met Pro Phe Glu Asp
145                 150                 155                 160

Ser Thr Phe Asp Val Ala Tyr Ala Ile Tyr Ser Leu Lys Tyr Ile Pro
                165                 170                 175

Asn Leu Asp Lys Val Met Lys Glu Ile Gln Arg Val Leu Lys Pro Gly
            180                 185                 190

Gly Lys Phe Ile Val Tyr Asp Leu Ile Lys Thr Asn Asp Tyr Asp Lys
      195                 200                 205

Asp Asn Lys Glu His Tyr Lys Thr Leu His His Leu Glu Tyr Ala Cys
    210                 215                 220

Gly Met Pro Ser Leu His Thr Gln Ser Glu Val Glu Ala Ala Ala Glu
225                 230                 235                 240

Lys Trp Glu Met Pro Val Val Glu Arg Glu Asn Leu Glu Glu Thr Tyr
                245                 250                 255

Gly Asn Arg Ala Phe His Tyr Cys Phe Ser Ala Ser Pro Met Phe Met
            260                 265                 270

Trp Leu Val Ser Ser Pro Val Ile Asp His Thr Ile Arg Met Ala Glu
      275                 280                 285

Ile Leu Arg Ile Leu Pro Ala Gly Phe Lys Gln Phe Asn Arg Thr Phe
    290                 295                 300

Leu Cys Gly Thr Val Asn Ser Ile Val Gly Gly Gly Arg Met Gly Ile
305                 310                 315                 320

Leu Ser Gly Ala Asp Ile Leu Leu Phe Glu Lys Lys Lys Ile
                325                 330

<210>  2
<211>  1005
<212>  DNA
<213>  Caenorhabditis elegans

<400>  2
atgtccatca atatgaatgc caactttttg aagcttctca cccattttcg gcgtcacgat      60

ttgacaaatt tcaagtcgga gcacgacacg ttgtacgaaa aagctctgga aactggagat     120

catttggaag tgacgtcaca ctattacagt gttatgagta ctgtaatcga tgagtatttt     180

ggcgggaatt ttcattttgt accgccgaaa ttcgaggggc aaaagcttga ggaggcgttg     240

aagtctcttc actgccacat tgctgaaaag ctggaattgt cggaaaatgt gcattgtctg     300

gatattggat gtggaattgg tggagttatg ctggatattg ctgatttcgg agccaagttg     360

acaggcgtca caattgctcc gaacgaggcg gagattggaa acgaaaaatt cgccaatatg     420

ggaatatcgg atcgttgtaa aattgtggcg gcagattgtc aaaaaatgcc ttttgaagat     480

tcgacatttg acgttgctta tgctatatat tccctgaaat atatcccgaa tttggacaaa     540

gttatgaagg aaattcagag agtgctcaag ccgggaggaa aatttattgt atacgatttg     600

ataaagacaa atgattatga caaggataat aaggaacatt ataagaccct ccatcatctg     660

gaatacgcgt gcggaatgcc atcacttcat acccaatcag aagtcgaagc ggccgccgaa     720

aaatgggaaa tgcctgtggt ggagcgggaa aatttggagg aaacctacgg aaatcgggct 780
tttcactact gtttctcggc gagcccaatg ttcatgtggt tggtgagcag tccagtgata 840
gatcacacta tccgaatggc tgaaattttg cgcatattac ccgccgggtt taaacaattt 900
aaccgaactt ttctgtgtgg aacagtgaac tcaattgtcg gaggaggacg gatgggaata 960
ctctccggag ccgatatttt gctgtttgag aagaagaaaa tctga 1005

## Description

The present invention relates to a compound for use as an anti-helminthic agent, said compound being selected from (i) a compound interfering with the transcription or translation of a nucleic acid sequence encoding a polypeptide having a function of introducing a methyl group at a specific position into the sterol nucleus of a sterol which nucleic acid sequence is (a) a nucleic acid sequence encoding a polypeptide comprising or consisting of the amino acid sequence of SEQ ID NO: 1; (b) a nucleic acid sequence comprising or consisting of the sequence of SEQ ID NO: 2; (c) a nucleic acid sequence which encodes a polypeptide which is at least 70% identical to the sequence of SEQ ID NO: 1;(d) the nucleic acid sequence of (b) wherein T is U; (e) a nucleic acid sequence hybridizing to the complementary strand of a nucleic acid molecule of any of (a) to (d); or (f) a nucleic acid sequence being degenerate to the sequence of the nucleic acid molecule of (d) or (e), wherein said compound is an antisense molecule, siRNA, shRNA, miRNA, aptamer, antibody or a ribozyme, and (ii) a compound interfering with the function of the polypeptide encoded by the nucleic acid sequence of (i) said compound being an antibody, enzyme, peptide or a small organic molecule.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Helminths are a division of eukaryotes (worms) including parasites living inside a host. They comprise cestodes, trematodes and nematodes. Nematodes are the most diverse phylum of pseudocoelomates, and one of the most diverse of all animals. Over 80,000 species have been described, of which over 15,000 are parasitic. The many parasitic forms include pathogens in most plants and animals (including humans). Parasitic nematodes are major challenges to human health and agriculture.

Plant parasitic nematodes include several groups causing severe crop losses. Several phytoparasitic nematode species cause histological damages to roots, including the formation of visible galls. Some nematode species transmit plant viruses through their feeding activity on roots. Other nematodes attack bark and forest trees. Plant parasitic nematodes cause an estimated 80 billion US dollars in crop damage annually. Some plant parasitic nematodes live outside the targeted plants.

Some 5,000 species of nematodes are estimated to be parasites of vertebrate animals and humans.

Parasitic nematodes, including whipworm, Ascaris, hookworm, and filarial worms, currently have infected about 3 billion people. The nematode parasites of humans cause a variety of disease conditions and symptoms, ranging from lack of energy and vigor to blindness and malformations. Pinworms, hookworms, and roundworms are extremely common intestinal helminth infections of humans; worldwide, roundworms are probably the most common.

The life cycle of parasitic nematodes is clinically important. One roundworm of note is *Caenorhabditis elegans*, which lives in the soil and has found much use as a model organism. The entire genome of *C. elegans* has been sequenced. Further, the developmental fate of every cell has been determined, and every neuron mapped. When exposed to unfavorable conditions, such as overcrowding or scarcity of food, *C. elegans* interrupts its reproductive life cycle and enters diapause by forming a specific dauer (enduring) larva (Riddle and Albert, 1997). Dauers have increased stress resistance due to reprogramming of gene expression, and similar changes have been found in long-living mutants (Golden and Melov, 2007). Dauer or dauer-like larval stages are also widely spread among parasitic nematodes and play an important role during infection (Viney, 2009). Thus, understanding the mechanisms of dauer regulation can shed light on general processes, like ageing, fertility or endocrine circuitry.

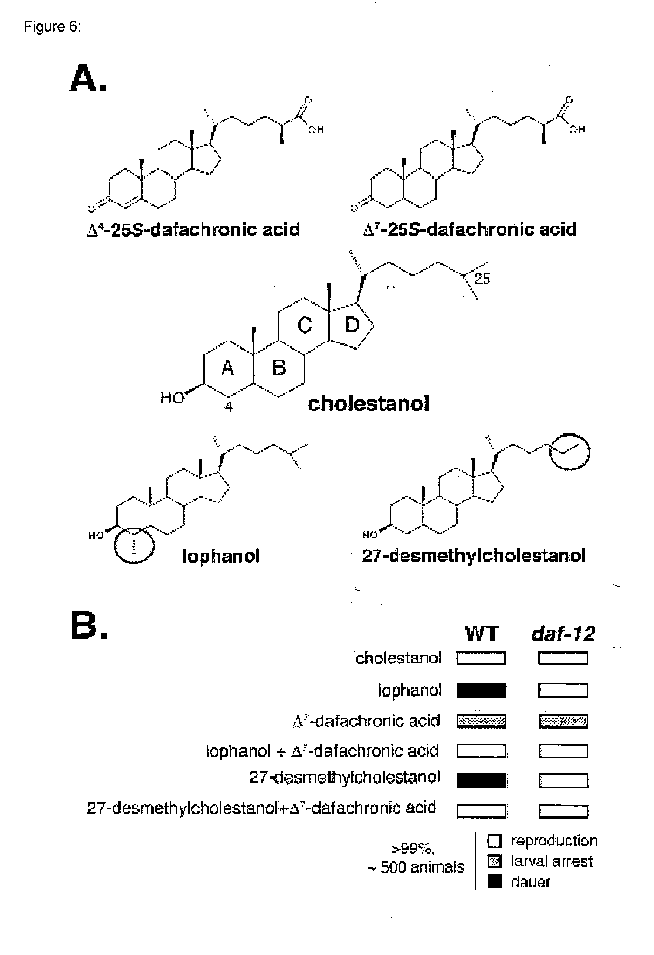

Biochemical studies and identification of genes involved in this process (*daf* genes, from dauer formation) revealed that dauer formation is initiated by specific pheromones (daumones/ short chain ascarosides (Butcher et al., 2007; Jeong et al., 2005); and is controlled by TGF-β, insulin and cyclic GMP signaling (Riddle and Albert, 1997). All these pathways converge on the activity of a nuclear hormone receptor, DAF-12 (Antebi et al., 1998; Antebi et al., 2000) which can bind sterol-derived, bile acid-like substances: dafachronic acids (DA) ((Motola et al., 2006) and Fig. 6A). Four isomers of DA have been described: two regioisomers, Δ⁴- and Δ⁷-DAs, each of which exist as 25R- and 25S-diastereomers (collectively referred to as DA). Some of the reactions for the production of DA have been clarified (Motola et al., 2006; Patel et al., 2008; Rottiers et al., 2006). The major step is performed by a cytochrome P450, DAF-9, which hydroxylates cholesterol twice in the C-26 position, producing a carboxyl group. In the presence of DA, the dauer promoting activity of DAF-12 is suppressed and worms remain in the reproductive cycle (Motola et al., 2006). In the absence of the hormone, i.e. in *daf-9* mutants, DAF-12 together with its co-repressor DIN-1 activates the diapause programme (Gerisch et al., 2001; Jia et al., 2002; Ludewig et al., 2004). Regulation of reproductive growth via DA and DAF-12 is a conserved process in nematodes as it is found in parasitic nematodes as well. In *Pristionchus pacificus* and *Strongyloides papillosus*, Δ⁷-DA can inhibit the L3 larval arrest required for production of infective larvae (Ogawa et al., 2009).

As mentioned above, the major regulators of dauer formation are TGF-β, insulin and cyclic GMP signaling pathways (Riddle and Albert, 1997). Down-regulation of TGF-β (DAF-7), or the insulin receptor (DAF-2), using thermo-sensitive loss of function alleles induces dauer formation at the restrictive temperature. As activation of the dauer programme via DAF-12 requires the absence of the dauer-repressing hormone, down-regulation of DAF-7 or DAF-2 presumably reduces levels of DA. This could be achieved by inhibition of its synthesis, by its degradation, or by its sequestration into an inactive form. Maintaining relatively low DA levels would allow a fast response to dauer-inducing pheromones. At present, the mechanism for hormone down-regulation is poorly understood.

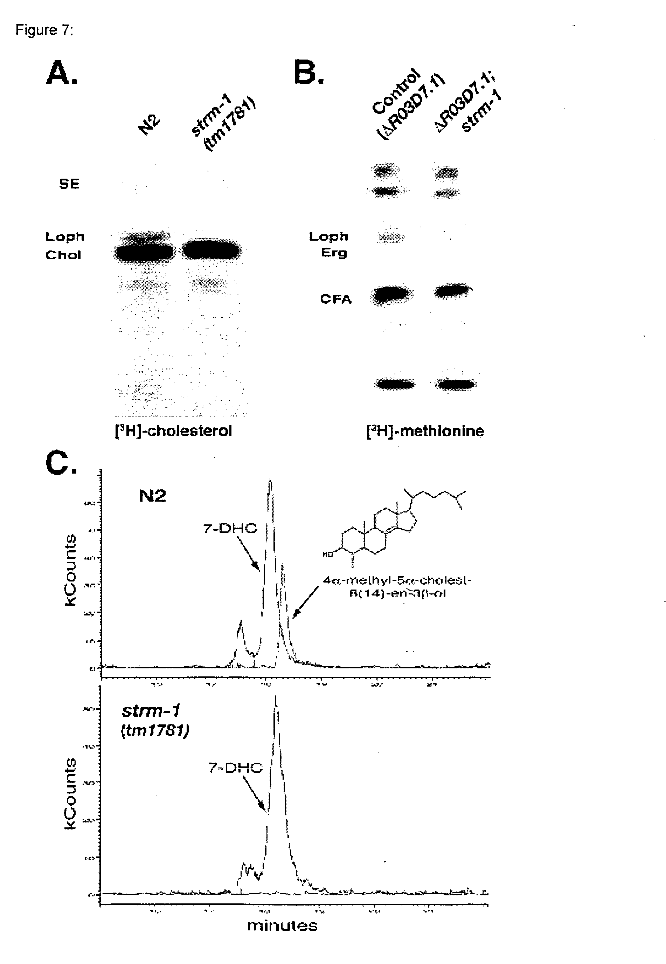

Nematodes are auxotrophic for sterols, i.e. they cannot synthesize sterols *de novo* and depend on their supply from food (Entchev and Kurzchalia, 2005; Kurzchalia and Ward, 2003). More than 20 years ago it was found that in contrast to other organisms (i.e. budding yeast, plants and mammals), *C. elegans* methylates the nucleus of sterols at the C-4 position (Chitwood et al., 1983). In organisms with *de novo* synthesis of sterols, the reverse reaction, demethylation of C-4 methylated precursors of cholesterol or ergosterol, is an essential step in sterol biosynthesis and involves more than ten enzymes. The importance of C-4 sterol methylation in nematodes remained enigmatic. A possible connection to dauer formation was first indicated when cholesterol in the food of worms was substituted with 4α-methylated-sterols (4-MS), lophenol or lophanol (Fig. 7A; Matyash et al., 2004), leading to dauer formation in the second generation. Therefore, to produce a dauer-repressing hormone, the C-4 position of sterols must be unmodified. A partially purified substance that rescued the lophenol effect was called gamravali (Matyash et al., 2004).

Dauer larvae resemble, in many aspects, infective larvae from parasitic nematodes (Viney, 2009). *Brugia malayi,* a filarial nematode causing lymphatic filariasis, enters the human host as an arrested L3 infective larva following a bite of the arthropod vector (Smith, 2000). Traveling through subcutaneous tissue for several days, the larva does not complete development through L4 to adulthood until it reaches the lymphatic system. This process must be tightly regulated, most likely by hormones. Recently, it was shown that the DA/DAF-12 system is conserved in parasitic nematodes and Δ⁷-DA can suppress formation of infective larvae and turn parasitic nematodes into free-living ones (Ogawa et al., 2009).

In sum, a lot of information has been compiled about the life circle and physiology of the model nematode *C. elegans*. This information, however, has not led to overall satisfactory results in the development of anti-helminthic agents as is evidenced, for example, by the enormous loss in crop yield referred to above. Accordingly, there is a need to develop further anti-helminthic agents. This need is addressed by the present invention.

Accordingly, the present invention relates to a compound for use as an anti-helminthic agent, said compound being selected from (i) a compound interfering with the transcription or translation of a nucleic acid sequence encoding a polypeptide having a function of introducing a methyl group at a specific position into the sterol nucleus of a sterol which nucleic acid sequence is (a) a nucleic acid sequence encoding a polypeptide comprising or consisting of the amino acid sequence of SEQ ID NO: 1; (b) a nucleic acid sequence comprising or consisting of the sequence of SEQ ID NO: 2; (c) a nucleic acid sequence which encodes a polypeptide which is at least 70% identical to the sequence of SEQ ID NO: 1;(d) the nucleic acid sequence of (b) wherein T is U; (e) a nucleic acid sequence hybridizing to the complementary strand of a nucleic acid molecule of any of (a) to (d); or (f) a nucleic acid sequence being degenerate to the sequence of the nucleic acid molecule of (d) or (e), wherein said compound is an antisense molecule, siRNA, shRNA, miRNA, aptamer, antibody or a ribozyme, and (ii) a compound interfering with the function of the polypeptide encoded by the nucleic acid sequence of (i) said compound being an antibody, enzyme, peptide or a small organic molecule.

The term "for use as an anti-helminthic agent" includes the term "for use in the treatment or prevention of infections by helminths". The helminths are preferably parasitic helminths throughout the embodiments of the invention.

The term "nucleic acid molecule", in accordance with the present invention, includes DNA, such as cDNA or, in a preferred embodiment genomic DNA, and RNA. It is understood that the term "RNA" as used herein comprises all forms of RNA including, in a preferred embodiment, mRNA. The term "nucleic acid molecule" is interchangeably used in accordance with the invention with the term "polynucleotide".

In those embodiments where the polynucleotide comprises (rather than consists of) the recited sequence, additional nucleotides extend over the specific sequence either on the 5' end or the 3' end or both. Those additional polynucleotides may be of heterologous or preferably homologous nature and may comprise stretches of about 50 to 500 nucleotides although higher or lower values are not excluded. In the case of homologous sequences, those embodiments preferably include complete chromosomes or genomes as long as the interfering compound interferes with the transcription or translation of the mentioned nucleic acid sequence. The case where the targeted nucleic acid sequence is comprised in the chromosome of the (life) helminth (rather than isolated) is a most preferred embodiment of the invention.

In connection with the invention, the term "nucleic acid sequence encoding a polypeptide having a function of introducing a methyl group at a specific position into the sterol nucleus of a sterol" is intended to mean a nucleic acid sequence which consists of or comprises a nucleic acid sequence encoding a polypeptide that covalently attaches said methyl group to one of several possible carbon atoms in one of the rings A, B, C or D of a sterol nucleus as shown in Scheme 1: 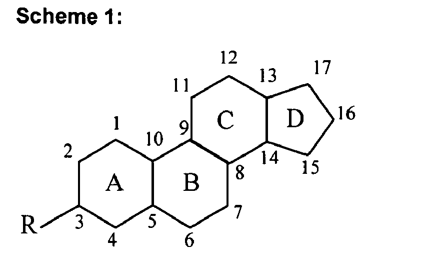 R = OH, O, S, NOH
A,B,C,D = are rings in which at least one bond can be unsaturated.

The nucleic acid sequence may also comprise regulatory regions or other untranslated regions. In other terms, the compound may target or interact with a regulatory region such as the natural promoter or another untranslated region (e.g. via the miRNA pathway or RNAi) to interfere with transcription or translation.

As mentioned, also encompassed by the present invention are nucleic acid sequences that encode polypeptides having at least 70% identity (such as at least 80%, more preferred at least 90%, even more preferred at least 95% such as at least 98% and most preferred at least 99% identity) at the amino acid level with the polypeptide depicted by SEQ ID NO: 1. It is to be understood that the comparison or alignment of sequences referred to herein means an alignment with the coding nucleotide sequence or encoded amino acid sequence. In one embodiment, the two sequences, when aligned, display the at least 70% identity over the same length, i.e. without either sequence extending N- or C-terminally over the other sequence. In the alternative, if such an extension occurs, it is preferred that it does not exceed more than 10 amino acids, more preferred not more than 5 amino acids over either terminus of the other sequence. The nucleic acid sequences include those that at least exhibit 70% identity with the above-recited nucleic acid sequences as defined in (a), (b), (c) and (d) supra. With increasing preference, the identity is at least 75%, at least 80%, at least 85%, at least 90%, at least 95%, at least 97,5% or at least 99%. Such molecules may be splice forms, homologous molecules from other species than *C. elegans,* such as orthologs, or mutated sequences from *C. elegans* to mention the most prominent examples. In accordance with the present invention, the term "at least % identical to" in connection with nucleic acid molecules describes the number of matches ("hits") of identical nucleic acids of two or more aligned nucleic acid sequences as compared to the number of nucleic acid residues making up the overall length of the amino acid sequences (or the overall compared part thereof). In other terms, using an alignment, for two or more sequences or subsequences the percentage of nucleic acid residues that are the same (e.g. at least 70% identity) may be determined, when the (sub)sequences are compared and aligned for maximum correspondence over a window of comparison, or over a designated region as measured using a sequence comparison algorithm as known in the art, or when manually aligned and visually inspected. Preferred nucleic acids in accordance with the invention are those where the described identity exists over a region that is at least 100 to 150 nucleotides in length, more preferably, over a region that is at least 200 to 400 nucleotides in length. More preferred nucleic acids in accordance with the present invention are those having the described sequence identity over the entire length of the nucleic acid molecule specifically identified by the sequence as described in (a) and (b) supra. Those having skill in the art will know how to determine percent sequence identity between/among sequences using, for example, algorithms such as those based on CLUSTALW computer programme (Thompson Nucl. Acids Res. 2 (1994), 4673-4680) or FASTA (Pearson and Lipman, Proc. Natl. Acad. Sci., 1988, 85; 2444), as known in the art. Although the FASTA algorithm typically does not consider internal non-matching deletions or additions in sequences, i.e., gaps, in its calculation, this can be corrected manually to avoid an overestimation of the % sequence identity. CLUSTALW, however, does take sequence gaps into account in its identity calculations. Also available to those having skill in this art are the BLAST and BLAST 2.0 algorithms (Altschul, Nucl. Acids Res., 1977, 25:3389). The BLASTN programme for nucleic acid sequences uses as default a word length (W) of 11, an expectation (E) of 10, M=5, N=4, and a comparison of both strands. For amino acid sequences, the BLASTP programme uses as default a word length (W) of 3, and an expectation (E) of 10. The BLOSUM62 scoring matrix (Henikoff, Proc. Natl. Acad. Sci., 1989, 89:10915) uses alignments (B) of 50, expectation (E) of 10, M=5, N=4, and a comparison of both strands. All those programmes may be used for the purposes of the present invention. However, preferably the BLAST programme is used. Accordingly, all the nucleic acid molecules having the prescribed function and further having a sequence identity of at least 70% as determined with any of the above recited or further programmes available to the skilled person and preferably with the BLAST programme fall under the scope of the invention.

The percentage sequence identity of polypeptide sequences can likewise be determined with programmes as the above explained programmes CLUSTLAW, FASTA and BLAST. Preferably the BLAST programme is used.

The term "hybridizing" as used in accordance with the present invention relates to stringent or non-stringent hybridization conditions. Preferably, it relates to hybridizations under stringent conditions. Said hybridization conditions may be established according to conventional protocols described, for example, in Sambrook, "Molecular Cloning, A Laboratory Manual", Cold Spring Harbor Laboratory (1989) N. Y., Ausubel, "Current Protocols in Molecular Biology", Green Publishing Associates and Wiley Interscience, N. Y. (1989), or Higgins and Hames (eds) "Nucleic acid hybridization, a practical approach" IRL Press Oxford, Washington DC, (1985). Hybridizing molecules or molecules falling under alternative (f), supra, also comprise fragments of the molecules identified in (a) or (b) wherein the nucleotide sequence need not be identical to its counterpart as defined in SEQ ID Nos 1 and 2, said fragments having a function as indicated above.

"Stringent conditions" refers to hybridization conditions under which the polynucleotides that are capable of hybridizing to the polynucleotides described above or parts thereof hybridize to these target sequences to a detectably greater degree than to other sequences (e.g., at least 2-fold over background). Stringent conditions are sequence-dependent and will be different in different circumstances. By controlling the stringency of the hybridization and/or washing conditions, target sequences that have at least 90% sequence identity, more preferably at least 95%, such as at least 98% and even 100% sequence identity to the probe can be identified (highly stringent hybridization conditions). Alternatively, stringency conditions can be adjusted to allow a higher degree of mismatching in sequences (low stringency conditions of hybridization). Such highly stringent and low stringent conditions for hybridization are well known to the person skilled in the art. For example, highly stringent conditions for hybridization comprise, e.g. an overnight incubation at 65°C in 4x SSC (600 mM NaCl, 60 mM sodium citrate) followed by washing at 65°C in 0.1x SSC for one hour. Alternatively, highly stringent hybridization conditions can comprise: an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing in e.g. 0.1-0.5x SSC at about 55-65°C for about 5 to 20 min. Also contemplated are nucleic acid molecules that hybridize to the specifically disclosed polynucleotides at lower stringency hybridization conditions ("low stringency conditions for hybridization"). Changes in the stringency of hybridization are primarily accomplished through the manipulation of formamide concentration (lower percentages of formamide result in lowered stringency), salt conditions, or temperature. For example, lower stringency conditions include an overnight incubation at 50°C in 4x SSC or an overnight incubation at 37°C in a solution comprising 6x SSPE (20x SSPE = 3M NaCl; 0.2M NaH₂PO₄; 0.02M EDTA, pH 7.4), 0.5% SDS, 30% formamide, 100 mg/ml salmon sperm blocking DNA; followed by washes at 50°C with 1x SSPE, 0.1% SDS. In addition, to achieve an even lower stringency, washes performed following stringent hybridization can be done at higher salt concentrations (e.g. 5x SSC). It is of note that variations in the above conditions may be accomplished through the inclusion and/or substitution of alternate blocking reagents. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado.

The term "being degenerate" as used in the invention relates to nucleic acid molecules the sequence of which differs from the nucleotide sequence of any of the above-described nucleic acid molecules due to the degeneracy of the genetic code.

The term "polypeptide" as used herein describes linear molecular chains of amino acids, including single chain proteins or their fragments, containing more than 30 amino acids and having the required methylase function. Polypeptides may further form oligomers consisting of at least two identical or different molecules. The corresponding higher order structures of such multimers are, correspondingly, termed homo- or heterodimers, homo- or heterotrimers etc. The term "polypeptide" also refers to naturally modified polypeptides where the modification is effected e.g. by glycosylation, acetylation, phosphorylation and similar modifications which are well known in the art.

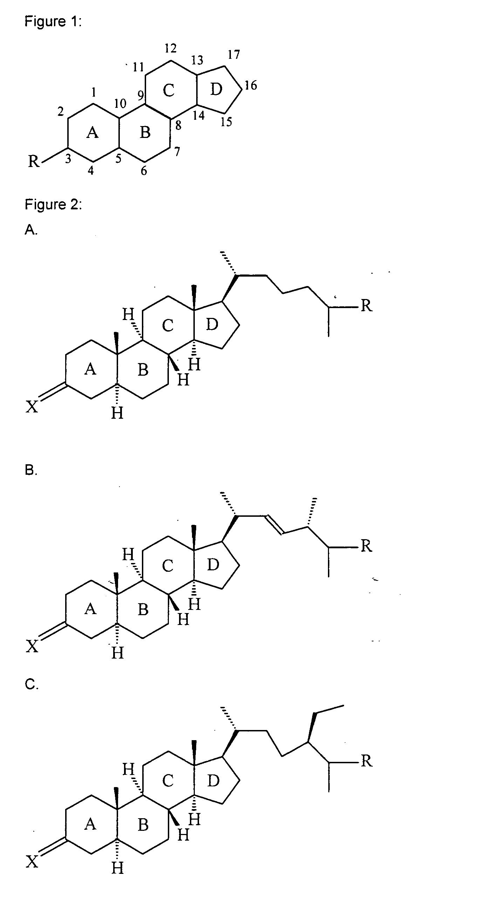

The term "sterol" in accordance with the present invention has the meaning as established in the art and denotes a compound comprising a sterol nucleus as shown in Scheme 1. It is preferred that the sterol nucleus as shown in Scheme 1 (Figure 1) has a planar molecular geometry. It is even more preferred that a planar sterol nucleus is modified in position C-17 of the sterol by a side chain comprising a modified or unmodified alkyl and in positions C-10 and C-13 by a methyl group, said positions being indicated in Scheme 1 (Figure 1).

The term "interfering with the transcription or translation" designates a compound which, in accordance with the present invention, specifically interacts with/specifically binds to the mentioned nucleic acid sequence including regulatory or untranslated regions thereof and lowers or abolishes the transcription and/or translation of the target nucleic acid sequence. The invention further relates to a compound interfering with the function of the polypeptide of the invention wherein said compound is an antibody, enzyme, peptide having up to 30 amino acids or a small organic molecule. Preferably, transcription and/or translation are reduced by at least 50%, more preferred at least 75% such as at least 90% or 95%, and even more preferred at least 98%.

The term "interfering with the function" is intended to mean that the compound interacts with and preferably specifically interacts with the polypeptide thus reducing or abolishing its function. Reduced function refers to decreasing the function of the described polypeptide by at least 25%, such as at least 50%, more preferred at least 75%, such as at least 85% or at least 95%. Thus, the above types of compounds can be characterized by performing one or more of the following effects: (1) the transcription of the gene encoding the protein to be inhibited is lowered, (2) the translation of the mRNA encoding the protein to be inhibited is lowered, (3) the protein performs its biochemical function with lowered efficiency in presence of the inhibitor, and (4) the protein performs its cellular function with lowered efficiency in presence of the inhibitor.

Compounds falling in class (1) include compounds interfering with the transcriptional machinery and/or its interaction with the promoter of said gene and/or with expression control elements remote from the promoter such as enhancers. Compounds of class (2) comprise antisense constructs and constructs for performing RNA interference well known in the art (see, e.g. Zamore (2001) or Tuschl (2001)). Generally, the following holds true: Targeting on the nucleic acid level is often effected by hybridisation to the target sequence. The higher the level of identity of the targeting compound to the complementary sequence of the target nucleic acid, the higher the level of specificity of targeting. Compounds of class (3) interfere with molecular function of the protein to be inhibited, in particular with the methylase activity. Accordingly, active site binding compounds, in particular compounds capable of binding to the active site of any methylase, are envisaged. More preferred are compounds specifically binding to an active site of the polypeptide. Also envisaged are compounds binding to or blocking substrate binding sites of the polypeptide as are compounds binding to or blocking binding sites of the polypeptide for other interaction partners. The latter group of compounds blocking binding sites of the polypeptide may be fragments or modified fragments with improved pharmacological properties of the naturally occurring binding partners. Class (4) includes compounds which do not necessarily directly bind to the polypeptide, but still interfere with the polypeptide activity, for example by binding to and/or inhibiting the function or inhibiting expression of members of a pathway which comprises the polypeptide. These members may be either upstream or downstream of the polypeptide within said pathway.

In a preferred embodiment, the compound is a small organic molecule. Small organic molecules are compounds of natural origin or chemically synthesized compounds. The term "small organic molecule" in accordance with the invention has the same meaning as in the art and preferably refers to molecules having a molecular weight less than 5000 g/mol, preferably less than 1000 g/mol, more preferably less than 750 g/mol and most preferably between 300 g/mol and 500 g/mol.

The efficiency of the inhibiting compound can be quantified by methods comparing the level of activity in the presence of the inhibitor to that in the absence of the inhibitor. For example, as an activity measure may be used: the change in amount of mRNA formed, the change in amount of protein formed, the change in amount of substrate converted or product formed, and/or the change in the cellular phenotype or in the phenotype of an organism. Preferably, said method is effected in high-throughput format. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits the expected activity, said mixture of test compounds may be de-convoluted to identify the one or more test compounds in said mixture giving rise to said activity.

In another preferred embodiment, the level of activity is less than 90%, more preferred less than 80%, 70%, 60% or 50% of the activity in absence of the inhibitor. Yet more preferred are inhibitors lowering the level down to less than 25%, less than 10%, less than 5% or less than 1% of the activity in absence of the inhibitor.

The term "antisense molecule" is known in the art and refers to a nucleic acid which is complementary to a target nucleic acid, usually an mRNA molecule. An antisense molecule according to the invention is capable of interacting with, more specifically hybridizing with the target nucleic acid. By formation of the hybrid, transcription of the target gene(s) and/or translation of the target mRNA is reduced or blocked. Standard methods relating to antisense technology have been described (see, e.g., Melani et al., Cancer Res. (1991) 51:2897-2901). The term "antisense molecule" in accordance with the present invention thus relates to a nucleic acid molecule, preferably an RNA molecule, that has a base sequence complementary to a given gene's messenger RNA (mRNA), i.e. the "sense" sequence. When the genetic sequence of a particular gene is known, it is possible to synthesize a strand of nucleic acid (DNA, RNA or a chemical analogue) that will bind to the mRNA produced by that gene and inactivate it, thus turning that gene "off' as mRNA has to be single stranded for it to be translated.

The term "siRNA" in accordance with the present invention refers to small interfering RNA, also known as short interfering RNA or silencing RNA. siRNAs are a class of 18 to 30, preferably 20 to 25, most preferred 21 to 23 or 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome. siRNAs have a well defined structure: a short double-strand of RNA (dsRNA), advantageously with at least one RNA strand having an overhang. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Thus, any gene of which the sequence is known can in principle be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Also preferably at least one RNA strand has a 5'- and/or 3'-overhang. Preferably, one or both ends of the double-strand has a 3'-overhang from 1-5 nucleotides, more preferably from 1-3 nucleotides and most preferably 2 nucleotides. In general any RNA molecule suitable to act as siRNA is envisioned in the present invention. The most efficient silencing was so far obtained with siRNA duplexes composed of 21-nt sense and 21-nt antisense strands, paired in a manner to have 2-nt 3'- overhangs. The sequence of the 2-nt 3' overhang makes a small contribution to the specificity of target recognition restricted to the unpaired nucleotide adjacent to the first base pair (Elbashir et al. Nature. 2001 May 24;411 (6836):494-8). 2'-deoxynucleotides in the 3' overhangs are as efficient as ribonucleotides, but are often cheaper to synthesize and probably more nuclease resistant.

The term "miRNA" as used herein refers to microRNAs, which are single-stranded RNA molecules of about 21-23 nucleotides in length, which regulate gene expression. miRNAs are encoded by genes that are transcribed from DNA but not translated into protein (non-coding RNA); instead they are processed from primary transcripts known as *pri-miRNA* to short stem-loop structures called *pre-miRNA* and finally to functional miRNA. Mature miRNA molecules are partially complementary to one or more messenger RNA (mRNA) molecules, and their main function is to downregulate gene expression. Animal miRNAs are usually complementary to a site in the 3' UTR whereas plant miRNAs are usually complementary to coding regions of mRNAs. Initial studies have indicated that miRNAs regulate gene expression post-transcriptionally at the level of translational inhibition at P-bodies in the cytoplasm. However, miRNAs may also guide mRNA cleavage in a manner similar to siRNAs.
miRNAs typically differ from siRNA because they are processed from single stranded RNA precursors and show only partial complementarity to mRNA targets. They have been implicated in a wide range of functions such as cell growth and apoptosis, development, neuronal plasticity and remodeling, and even insulin secretion. miRNA can also be used to silence gene expression via RNA interference.

A "shRNA" in accordance with the present invention is a short hairpin RNA, which is a sequence of RNA that makes a (tight) hairpin turn that can also be used to silence gene expression via RNA interference. shRNA preferably utilizes the U6 promoter for its expression. The shRNA hairpin structure is cleaved by the cellular machinery into siRNA, which is then bound to the RNA-induced silencing complex (RISC). This complex binds to and cleaves mRNAs which match the shRNA that is bound to it.
siRNAs and shRNAs of the present invention are preferably chemically synthesized using appropriately protected ribonucleoside phosphoramidites and a conventional RNA synthesizer. Suppliers of RNA synthesis reagents include Proligo (Hamburg, Germany), Dharmacon Research (Lafayette, CO, USA), Pierce Chemical (part of Perbio Science, Rockford, IL , USA), Glen Research (Sterling, VA, USA), ChemGenes (Ashland, MA, USA), and Cruachem (Glasgow, UK).

Pharmaceutical compositions of the invention may comprise an antisense molecule, siRNA, shRNA or miRNA. The ability of antisense molecules, siRNA, shRNA and miRNA to potently, but reversibly, silence genes *in vivo* makes these molecules particularly well suited for use in a pharmaceutical composition of the invention. Ways of administering siRNA to humans are described in De Fougerolles et al., Current Opinion in Pharmacology, 2008, 8:280-285. Accordingly, such pharmaceutical compositions may be administered directly formulated as a saline, via liposome based and polymer-based nanoparticle approaches, as conjugated or complexation pharmaceutical compositions, or via viral delivery systems. Direct administration comprises injection into tissue, intranasal and intratracheal administration. Liposome based and polymer-based nanoparticle approaches comprise the cationic lipid Genzyme Lipid (GL) 67, cationic liposomes, chitosan nanoparticles and cationic cell penetrating peptides (CPPs). Conjugated or complexation pharmaceutical compositions comprise PEI-complexed antisense molecules, siRNA, shRNA or miRNA. Further, viral delivery systems comprise influenza virus envelopes and virosomes.

The term "aptamer" in accordance with the present invention refers to DNA or RNA molecules that have been selected from random pools based on their ability to bind other molecules. Aptamers have been selected which bind nucleic acid, proteins, small organic compounds, and even entire organisms. A database of aptamers is maintained at http://aptamer.icmb.utexas.edu/.

More specifically, aptamers can be classified as DNA or RNA aptamers or peptide aptamers. Whereas the former consist of (usually short) strands of oligonucleotides, the latter consist of a short variable peptide domain, attached at both ends to a protein scaffold. Nucleic acid aptamers are nucleic acid species that have been engineered through repeated rounds of *in vitro* selection or equivalently, SELEX (systematic evolution of ligands by exponential enrichment) to bind to various molecular targets such as small molecules, proteins, nucleic acids, and even cells, tissues and organisms. Peptide aptamers are proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable loop length is typically comprised of 10 to 20 amino acids, and the scaffold may be any protein which have good solubility properties. Currently, the bacterial protein Thioredoxin-A is the most used scaffold protein, the variable loop being inserted within the reducing active site, which is a - Cys-Gly-Pro-Cys- loop in the wild protein, the two cysteins lateral chains being able to form a disulfide bridge. Peptide aptamer selection can be made using different systems, but the most used is currently the yeast two-hybrid system.

Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as *in vivo* diagnostic imaging. Several modifications, such as 2'-fluorine-substituted pyrimidines, polyethylene glycol (PEG) linkage, etc. are available to scientists with which the half-life of aptamers easily can be increased to the day or even week time scale.

The term "antibody", in accordance with the present invention, comprises polyclonal and monoclonal antibodies as well as derivatives or fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The antibody of the invention also includes embodiments such as chimeric, single chain and humanized antibodies, as well as antibody fragments, like, inter alia, Fab fragments, as well as fusion proteins consisting of Eph, ephrin or phosphatase extracellular domains and Fc. Antibody fragments or derivatives further comprise Fab, F(ab')₂, Fv or scFv fragments; see, for example, Harlow and Lane (1988) and (1999), loc. cit. Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies specific for polypeptide(s) and fusion proteins of this invention. Also, transgenic animals may be used to express humanized antibodies specific for polypeptides and fusion proteins of this invention. Most preferably, the antibody of this invention is a monoclonal antibody. For the preparation of monoclonal antibodies, any technique, which provides antibodies produced by continuous cell line cultures, can be used. Examples for such techniques include the original hybridoma technique (Köhler and Milstein (1975) Nature 256, 495) as further developed by the art, the trioma technique, the human B-cell hybridoma technique (Kozbor (1983) Immunology Today 4, 72) and the EBV-hybridoma technique to produce human monoclonal antibodies (Cole et al. (1985) Monoclonal Antibodies and Cancer Therapy, Alan R. Liss, Inc., 77). Surface plasmon resonance as employed in the BlAcore system can be used to increase the efficiency of phage antibodies, which bind to an epitope of a polypeptide of the invention (Schier (1996) Human Antibodies Hybridomas 7, 97; Malmborg (1995) J. Immunol. Methods 183, 7). It is also envisaged in the context of this invention that the term "antibody" comprises antibody constructs, which may be expressed in cells, e.g. antibody constructs which may be transfected and/or transduced via, amongst others, viruses or plasmid vectors. The antibody described in the context of the invention is capable to specifically bind/interact with an epitope of the mentioned polypeptide, preferably STRM-1, STRM-1 being defined further below. The term "specifically binding/interacting with" as used in accordance with the present invention means that the antibody does not or essentially does not cross-react with an epitope of similar structure. Cross-reactivity of a panel of antibodies under investigation may be tested, for example, by assessing binding of said panel of antibodies under conventional conditions to the epitope of interest as well as to a number of more or less (structurally and/or functionally) closely related epitopes. Only those antibodies that bind to the epitope of interest in its relevant context (e.g. a specific motif in the structure of a protein) but do not or do not essentially bind to any of the other epitope are considered specific for the epitope of interest and thus to be antibodies in accordance with this invention (the same holds true, *mutatis mutandis*, for the specificity of aptamers). Corresponding methods are described e.g. in Harlow and Lane, 1988 and 1999, loc cit. The antibody specifically binds to/interacts with conformational or continuous epitopes, which are unique for the mentioned polypeptide, preferably STRM-1. A conformational or discontinuous epitope is characterized for polypeptide antigens by the presence of two or more discrete amino acid residues which are separated in the primary sequence, but come together on the surface of the molecule when the polypeptide folds into the native protein/antigen (Sela (1969) Science 166, 1365; Laver (1990) Cell 61, 553). The two or more discrete amino acid residues contributing to the epitope are present on separate sections of one or more polypeptide chain(s). These residues come together on the surface of the molecule when the polypeptide chain(s) fold(s) into a three-dimensional structure to constitute the epitope. In contrast, a continuous or linear epitope consists of two or more discrete amino acid residues, which are present in a single linear segment of a polypeptide chain. Antobodies may be administered for example as described in WO/2000/029019 and US 6,294,171.

The term "ribozyme" refers to ribonucleic acid enzyme, also called RNA enzyme or catalytic RNA, that are RNA molecules that are capable of catalysing chemical reactions. Many naturally occurring ribozymes catalyse either their own cleavage or the cleavage of other RNAs, but they have also been found to catalyse the aminotransferase activity of the ribosome. Examples of well-characterized small self-cleaving RNAs are the hammerhead, hairpin, hepatitis delta virus, and in vitro-selected lead-dependent ribozymes. The principle of catalytic self-cleavage is now well established. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site.

The basic principle of constructing hammerhead ribozymes is as follows: An interesting region of the RNA, which contains the GUC (or CUC) triplet, is selected. Two oligonucleotide strands, each with 6 to 8 nucleotides, are taken and the catalytic hammerhead sequence is inserted between them. Molecules of this type were synthesized for numerous target sequences. They showed catalytic activity in vitro and in some cases also in vivo. The best results are usually obtained with short ribozymes and target sequences.

The enzyme for inhibiting the function of the polypeptide of the invention refers to an enzymes which lower or abolish the activity of the polypeptide of the invention, preferably STRM-1, such as proteases or peptidases.

In accordance with the present invention, a nucleic acid molecule encoding a methylase that methylates a specific position within the sterol nucleus of a sterol, more specifically and preferred the C-4-position of cholesterol was surprisingly identified as a target for the prevention or treatment of helminthic infections, more preferably, of nematode infections. In accordance with the invention this preferred methylase is termed STRM-1 (STerol A-Ring Methylase-1). A particularly preferred embodiment is represented by amino acid SEQ ID No 1. The methylase uses the biosynthetic precursors of DAs as substrates. It has been also found that in the absence of DAF-9 4-MS were elevated. Thus, STRM-1 regulates dauer formation by reducing the amounts of DA through modifying the substrate(s) of DAF-9 to make them unsuitable for DA generation. Consistent with this, dauer constitutive (*daf-c*) mutants, like the TGF-β mutant *daf-7(e1372)* were more sensitive to exogenous DA in a *strm-1(tm1781)* mutant background, and *strm-1(tm1781)* animals had elevated levels of DA. As a result, several *daf-c* mutants showed a decreased frequency of dauer formation and related phenotypes when *strm-1* was deleted. Moreover, depletion of STRM-1 results in a dramatic effect under more physiological conditions: animals become almost insensitive to dauer-inducing pheromones. Thus, methylation of the sterol nucleus by STRM-1 maintains low levels of DA to facilitate regulation of dauer larva formation.

The data show that the activity of STRM-1 is directly associated with the process of dauer larva formation and regulation of the amount of DA. By modifying the substrates that are used for the synthesis of DA, STRM-1 can reduce the amount of hormone produced. Loss of SRTM-1 function leads to elevated levels of DA and inefficient dauer formation. Importantly, sterol methylation was not previously recognized as a mechanism for regulating hormone activity. Moreover, the C-4 sterol nucleus methylation catalyzed by STRM-1 occurs in accordance with the present invention in nematodes.

Thus, the polypeptide described above and in particular STRM-1 is a highly promising target for therapeutic strategies to combat parasitic helminthic, and in particular nematode infections. As explained herein above, it may be targeted at either the nucleic acid or the polypeptide level employing targeting techniques which are as such well known in the art. The helminthic infections give rise to a variety of dieseases, both in the plant or animal kingdom. These diseases can be prevented or treated with the compound to be used in accordance with the invention. Such diseases include in animals Enterocolitis, Cholangitis, Hepatocholangitis, Pancreatitis, Choloncarcinoma, Pneumopathia Bilharzia, Vitamin B12 deficiency Anaemia, Enteropathia, Cysticerosis, Echinococcosis, Trichinosis, Enteropathia, anal itching, mine workers helminthiasis, transient eosinophil Pneumonia, Appendictis, Lymphoedema, Elephantitis, Loiasis, Ochnoceraciasis, skin rash. Nematode infections in humans include preferably ascariasis, trichuriasis, hookworm, enterobiasis, strongyloidiasis, filariasis, and trichinosis.

In plants the helminthic infections may give rise to root injury or yellowing and eventual loss of foliage. Moreover, new flushes of growth may be stunted and weak, with fewer and smaller leaves than healthy plants. Infected plants tend to wilt more readily during low water or drought conditions than uninfected plants. Plant nematodes are classified according to their feed behaviour as follows: migratory ectoparasites (e.g., dagger nematode, *Xiphinema* spp.), sedentary ectoparasites (e.g., citrus nematode, *Tylenchulus semipenetrans*), migratory endoparasites (e.g., root-lesion nematode, *Pratylenchus* spp.) and sedentary endoparasites (e.g., root-knot nematodes, *Meloidogyne* spp. and cyst nematode, *Heterodera* spp.).

Furthermore, to the inventors' knowledge, STRM-1 is the first methylase that is capable of introducing a methyl group at a specific position in the sterol nucleus and not its side chain.

In a preferred embodiment of the invention, the polypeptide has a function of introducing a methyl group at a specific position into the A-ring (position C-1, C-2, C-4, C-5 and C-10) of the sterol nucleus of a sterol, as shown in Scheme 1.

In a particularly preferred embodiment of the compound of the invention, the polypeptide has a function of introducing a methyl group into position C-4 of the A-ring of a sterol, said A-ring being indicated in Scheme 1.

In a further preferred embodiment of the compound of the invention, the sterol is a cholesterone, cholesterol, ergosterol, ergosterone, sitosterol or sitosterone.

The terms "cholesterone or cholesterol" (depending on the substituent X) (Scheme 2A), "ergosterol or ergosterone" (depending on the substituent X) (Scheme 2B) and sitosterol or sitosterone" (depending on the substituent X) (Scheme 2C) in accordance with the present invention relate to the compounds as shown in Scheme 2. 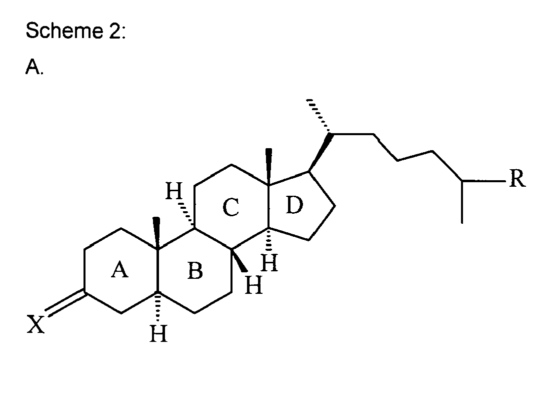 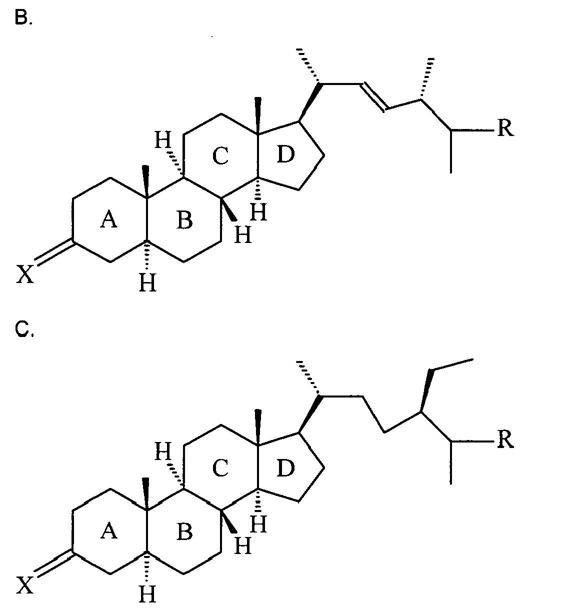 A-C:
X = OH, O, S, NOH
R = CH₃, OH, O, S, COOH, CONH₂
A,B,C,D = are rings in which at least one bond can be unsaturated

It is preferred that the compounds in Scheme 2 are naturally occurring cholesterone, cholesterol, ergosterol, ergosterone, sitosterol and sitosterone. All these compounds are well known in the art and their chemical formulae, for example, available from textbooks as, for example, "Lehrbuch der organischen Chemie" (23rd edition, S.Hirzel Verlag) or the Chemical Abtstract Databae (www.cas.org).

In a particularly preferred embodiment of the invention, the sterol as shown in Scheme 2A is dafachronic acid as shown in Scheme 3. 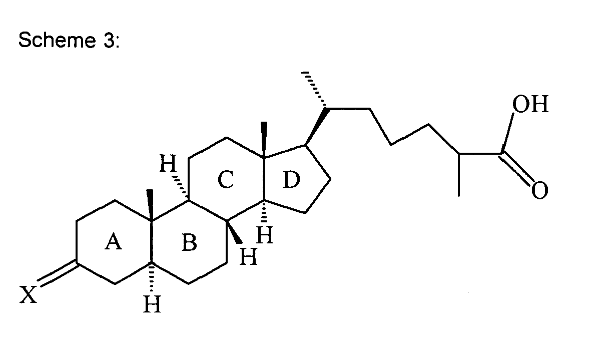 X = OH, O, S, NOH
A,B,C,D = are rings in which at least one bond can be unsaturated

In a further particularly preferred embodiment of the invention, the sterol as shown in Scheme 3 is Δ⁴⁻ dafachronic acid (Scheme 4A) or Δ⁷-dafachronic acid (Scheme 4B), Δ⁸⁽¹⁴⁾-dafachronic acid (Scheme 4C) or saturated dafachronic acid (Scheme 4D) as shown in Scheme 4.

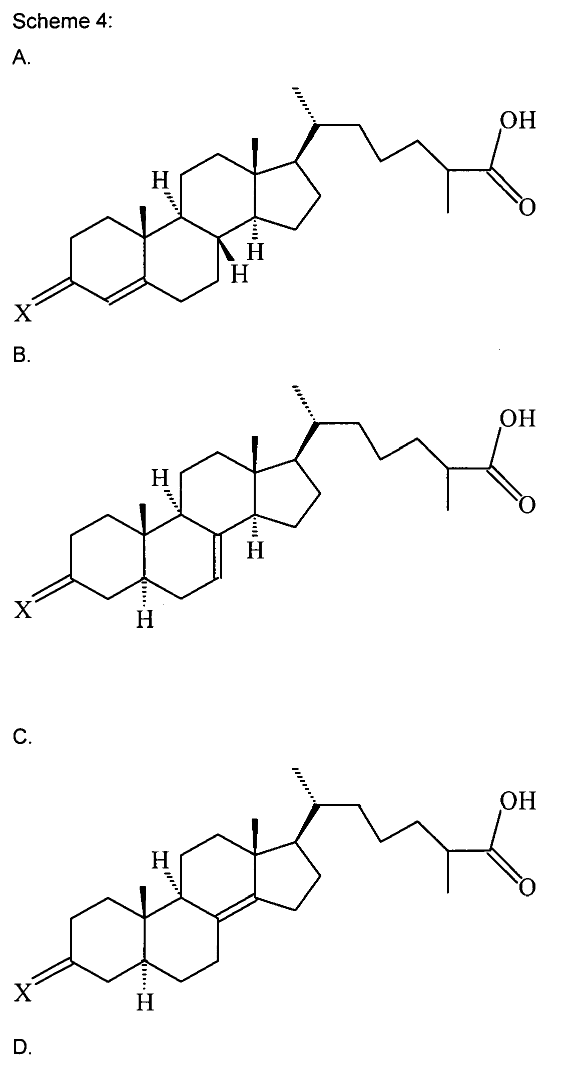

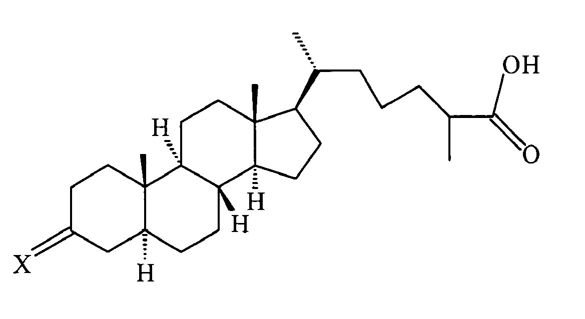

A preferred embodiment of the invention relates to a small organic molecule for inhibiting the function of the polypeptide as defined in the present invention selected from the formulae in Scheme 5 A-E. 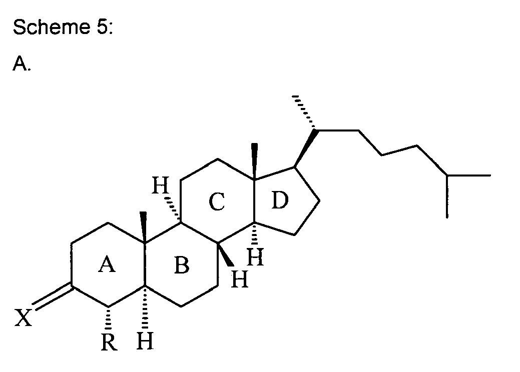 X = O, S, NOH;
R = H, C≡CH, C≡N, N=N⁺X⁻, N₃, COOH, NO₂, Cl, Br, I;
A,B,C,D = are rings in which at least one bond can be unsaturated 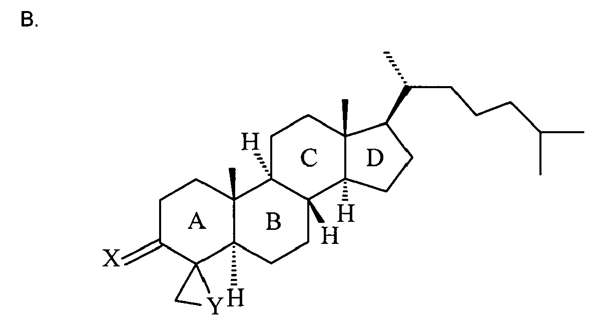 X = O, S, NOH;
Y = O, S, NR (R = alkyl, benzyl, aryl);
A,B,C,D = are rings in which at least one bond can be unsaturated 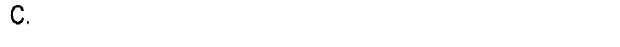 C. 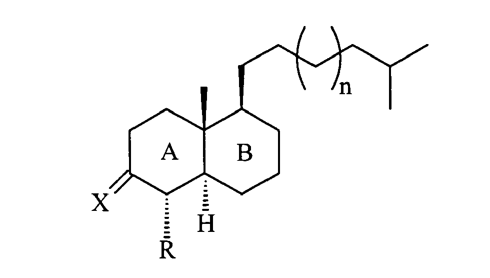 n = 0 - 9;
X = O, S, NOH;
R = H, C=CH, C=N, N=N⁺X⁻, N₃, COOH, NO₂, Cl, Br, I;
A,B = are rings in which at least one bond can be unsaturated 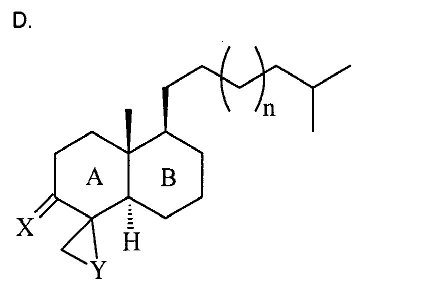 n = 0 - 9;
X = O, S, NOH;
Y = O, S, NR (R = alkyl, benzyl, aryl);
A,B = are rings in which at least one bond can be unsaturated 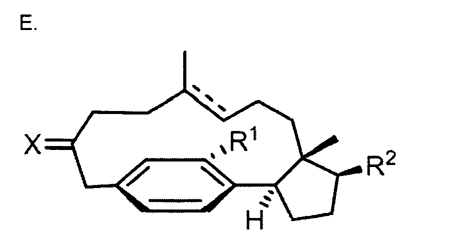 X = O, S, NOH
R¹ = CHO, CH₃, CH₂OH, COOH, COOMe, COMe
R² = CH(CH₃)CH₂CH₂CH₂CH(CH₃)₂, CH(CH₃)CH=CHCH(CH₃)CH(CH₃)₂

Preferred small organic molecules of the invention can be prepared according to the guidelines provided in Martin et al. 2008, Martin et al. 2009, Martin et al., 2009 and Martin et al., 2009.

Another embodiment of the invention relates to a plant protective agent comprising the compounds as defined by the invention. The compound is, as mentioned, useful in targeting the nucleic acid molecule of the invention or interfering with the function of the polypeptide of the invention.

The term "plant-protective agent" relates to agents, preferably contained in a composition used in the prevention or treatment of diseases related to plants. Formulations of plant-protective compositions comprise wettable powders (WPs) or micogranules for,use e.g., in a plant powder, seed powder or soil additive, and emulsifiable concentrates (ECs). Micogranules are fine droplets of the plant protective agent in a polymer capsule. Formation of the capsule is preferably achieved by polymerisation of the polymer in presence of the fine distributed plant protective agent. The plant protective agent can diffuse slowly from this capsule and is therefore effective for a long time. Being liquids, the EC compositions are easier to handle, their portioning can be handled by a simple volumetric measurement. The biological activity of the EC compositions is usually higher than that of the WP compositions. EC compositions can be prepared only from an active ingredient which is liquid or, when a solvent can be found in which the active ingredient can be dissolved to give a solution of 10 to 85% concentration (depending on the usual concentrations of the application) without any risk of the interim alteration of the active ingredient. EC compositions usually contain a high amount of solvent. The drawback of the EC compositions can be diminished by formulating the active ingredient in an emulsifiable microemulsion concentrate. Microemulsion is a colloidal system which, in a first approach, differs from a true emulsion in the dimension of its particles which are smaller by an order of magnitude than those of a true emulsion. According to the general definition, this system contains surface active agents and two immiscible liquids, one of them is usually water, alcohol, a lipophylic solvent or a mixture of solvents, though, in principle, it is also possible to prepare a microemulsion by using another solvent. The surfactant may be the mixture of even 6 to 8 tensides and additionally, it may contain alcohols or amines of medium chain length as auxillary surfactants (co-surfactants). A "suitable carrier" in connection with the plant protective composition usually is a non-toxic solid, semisolid or liquid filler, diluent, encapsulating material or formulation auxiliary of any type. Examples of suitable carriers are well known in the art and comprise water or organic solvents as liquids. The suitable composition comprising the inhibitors to the polypeptides of the invention as plant protective composition can be readily determined by the skilled person using methods well-known in the art. Non limiting examples of such methods are the treatment of plants or plant tissues with the inhibiting compound of interest and the subsequent infection with helminths and analysing the protective effect of the inhibiting compound as compared to control experiments in un-protected plants and plant tissues (see for example Alan and Earle, 2002). Also the production of transgenic plants as such expressing the peptide of the invention is well known to the person skilled in the art (Aerts *et al*., 2007; Oard and Enright, 2006). Such transgenic plants or parts thereof may be infected with helminths and analysed for the protective effect of the inhibitor as compared to control experiments in wild-type plants. The plant protective agent may also be used as plant protective additive in seed material, plant material, soil material, as e.g. in flower soil, greenhouse soil or other soil.

In a preferred embodiment the plant is infected by a plant parasitic nematode and the disease caused or enhanced thereby. The plant may preferably be any of the ones mentioned herein above. The plant protective agent is thus preferably an anti-helminthic (with the definition of helminthes provided herein above), preferably an anti-nematode agent. Plant parasitic nematodes include all nematodes causing severe crop losses. Such nematodes are but are not limited to *Aphelenchoides* (foliar nematodes), *Ditylenchus, Globodera* (potato cyst nematodes), *Heterodera* (soybean cyst nematodes), *Longidorus, Meloidogyne* (root-knot nematodes), *Nacobbus, Paratrichodorus Pratylenchus* (lesion nematodes), *Trichodorus* (stubby-root nematode), *Bursaphelenchinae* (pine wood nematode), and *Xiphinema* (dagger nematodes). Several phytoparasitic nematode species cause histological damages to roots, including the formation of visible galls (e.g. by root-knot nematodes). Some nematode species transmit plant viruses through their feeding activity on roots. One of them is *Xiphinema index*, vector of GFLV (Grapevine Fanleaf Virus), an important disease of grapes. Other nematodes attack bark and forest trees. The most important representative of this group is *Bursaphelenchus xylophilus*, the pine wood nematode.

A further embodiment of the invention relates to a pharmaceutical composition comprising a compound as defined by the invention. The compound is, as mentioned, useful in targeting the nucleic acid molecule as described or interfering with the function of the polypeptide as described. The pharmaceutical composition may, just as the plant protective agent, be used in the treatment or prevention of helminth infections.

It is preferred that the pharmaceutical composition is an anti-helminthic, preferably an anti-nematode pharmaceutical composition. The pharmaceutical composition is preferably administered to mammals such as domestic and pet animals (see also below). Most preferred it is administered to humans. The pharmaceutical compositions described herein can be administered to the subject at a suitable dose. Administration of the suitable compositions may be effected by different ways, e.g., by intravenous, intraperitoneal, subcutaneous, as well as transdermal administration.

The pharmaceutical composition for use in accordance with the present invention can be formulated in conventional manner according to methods found in the art, using one or more physiological carriers or excipient, see, for example Ansel et al., "Pharmaceutical Dosage Forms and Drug Delivery Systems", 7th edition, Lippincott Williams & Wilkins Publishers, 1999. The pharmaceutical composition may, accordingly, be administered orally, parenterally, such as subcutaneously, intravenously, intramuscularly, intraperitoneally, intrathecally, transdermally, transmucosally, subdurally, locally or topically via iontopheresis, sublingually, by inhalation spray, aerosol or rectally and the like in dosage unit formulations optionally comprising conventional pharmaceutically acceptable excipients.

For oral administration, the pharmaceutical composition of the invention can take the form of, for example, tablets or capsules prepared by conventional means with pharmaceutical acceptable excipients such as binding agents (e.g., pregelatinised maize starch, polyvinylpyrrolidone, hydroxypropyl methylcellulose), fillers (e.g., lactose, microcrystalline cellulose, calcium hydrogen phosphate), lubricants (e.g., magnesium stearate, talc, silica), disintegrants (e.g., potato starch, sodium starch glycolate), or wetting agents (e.g., sodium lauryl sulphate). The pharmaceutical composition can be administered with a physiologically acceptable carrier to a patient, as described herein. In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium ion, dried skim milk, glycerol, propylene, glycol, water, ethanol and the like. The composition, if desired, can also contain minor amounts of wetting or emulsifying agents, or pH buffering agents. These compositions can be in the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the aforementioned compounds, preferably in purified form, together with a suitable amount of carrier so as to provide the form for proper administration to the patient. The formulation should suit the mode of administration.

Liquid preparations for oral administration can be in the form of, for example, solutions, syrups, or suspensions, or can be presented as a dry product for constitution with water or other suitable vehicle before use. Such liquid preparation can be prepared by conventional means with pharmaceutically acceptable additives such as suspending agents (e.g., sorbitol, syrup, cellulose derivatives, hydrogenated edible fats), emulsifying agents (e.g., lecithin, acacia), non-aqueous vehicles (e.g., almond oil, oily esters, ethyl alcohol, fractionated vegetable oils), preservatives (e.g., methyl or propyl-p-hydroxycarbonates, soric acids). The preparations can also contain buffer salts, flavouring, coloring and sweetening agents as deemed appropriate. Preparations for oral administration can be suitably formulated to give controlled release of the pharmaceutical composition of the invention.

For administration by inhalation, the pharmaceutical composition of the invention is conveniently delivered in the form of an aerosol spray presentation from a pressurised pack or a nebulizer, with the use of a suitable propellant (e.g., dichlorodifluoromethane, trichlorofluoromethane, dichlorotetrafluoroethane, carbon dioxide or other suitable gas). In the case of a pressurised aerosol, the dosage unit can be determined by providing a valve to deliver a metered amount. Capsules and cartridges of, for example, gelatine, for use in an inhaler or insufflator can be formulated containing a powder mix of the pharmaceutical composition of the invention and a suitable powder base such as lactose or starch.

The pharmaceutical composition of the invention can be formulated for parenteral administration by injection, for example, by bolus injection or continuous infusion. Site of injections include intra-venous, intra-peritoneal or sub-cutaneous. Formulations for injection can be presented in units dosage form (e.g., in phial, in multi-dose container), and with an added preservative. The pharmaceutical composition of the invention can take such forms as suspensions, solutions or emulsions in oily or aqueous vehicles, and can contain formulatory agents such as suspending, stabilizing, or dispersing agents. Alternatively, the agent can be in powder form for constitution with a suitable vehicle (e.g., sterile pyrogen-free water) before use. Typically, compositions for intravenous administration are solutions in sterile isotonic aqueous buffer. Where necessary, the composition can also include a solubilizing agent and a local anesthetic such as lignocaine to ease pain at the site of the injection. Generally, the ingredients are supplied either separately or mixed together in unit dosage form, for example, as a dry lyophilised powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients can be mixed prior to administration.

The pharmaceutical composition of the invention can be formulated for transdermal administration. Transdermal compositions are typically formulated as a topical ointment or cream containing the active ingredient(s), generally in an amount ranging from about 0.01 to about 20% by weight, preferably from about 0.1 to about 20% by weight, preferably from about 0.1 to about 10% by weight, and more preferably from about 0.5 to about 15% by weight. When formulated as a ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or the formulation. All such known transdermal formulations and ingredients are included within the scope of this invention. The compounds of this invention can also be administered by a transdermal device. Accordingly, transdermal administration can be accomplished using a patch either of the reservoir or porous membrane type, or of a solid matrix variety.

The pharmaceutical composition of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The pharmaceutical composition of the invention can also, if desired, be presented in a pack, or dispenser device which can contain one or more unit dosage forms containing the said agent. The pack can for example comprise metal or plastic foil, such as blister pack. The pack or dispenser device can be accompanied with instruction for administration.

The pharmaceutical composition of the invention can be administered as sole active agent or can be adminstered in combination with other agents.

The pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.

A further embodiment of the invention relates to a compound of the invention for use in an anti-helminthic treatment.

The term "anti-helminthic treatment" in accordance with the invention preferably refers to the treatment of a subject infected by an animal parasitic nematode, in particular for treating a disease caused by infection of a subject with a nematode.

For example, the following sterol-derived signaling can be affected in the nematode by the anti-helminthic treatment: Gonad development, fertility, growth, molting, general lipid homeostasis, and dauer-larval-stage or dauer-like-larval-stage formation.

The term "subject" in accordance with the invention refers to an animal which can be preferably a vertebrate, more preferably a mammal and most preferably a domestic animal or a pet animal such as horse, cattle, pig, sheep, goat, dog or cat, and most preferably a human. Also, the subject may be a fish or bird such as a chicken.

Animal parasitic nematodes include but are not limited to ascarids (*Ascaris*), hookworms, pinworms (*Enterobius*), whipworms (*Trichuris trichiura*), Pristinochus (e.g., *Pristionchus pacificus*), Strongyluides (e.g., *Strongyloides papillosus*) and filarids (e.g., *Wuchereria bancrofti, Brugia timori, Brugia malayi*). The species *Trichinella spiralis*, commonly known as the trichina worm, occurs e.g. in rats, pigs, and humans, and is responsible for the disease trichinosis. *Baylisascaris* usually infests wild animals but can be deadly to humans as well. *Haemonchus contortus* is one of the most abundant infectious agents in sheep around the world, causing great economic damage to sheep farms.

The invention furthermore relates to a multi-cellular organism carrying (a) the nucleic acid sequence as defined in accordance with the invention or (b) a vector comprising the nucleic acid sequence as defined in accordance with the invention. Said multi-cellular organism may be either a plant or a non-human animal. The multi-cellular organism (also referred to as transgenic multi-cellular organism because of the presence of the referenced nucleic acid sequence), when being an animal, is preferably a nematode and more preferred *C. elegans*. The animal may also be a vertebrate and more preferred a mammal such as pig, sheep, dog, cat, cow or goat.

If a multi-cellular organism is a plant, it may be a monocotyl or a dicotyl. Preferred examples of plants are domesticated plants such as vegetable, crops, forest trees and fruits, or ornamental plants typically grown in the garden. The plant is preferably selected independently from *Anacardium, Anona, Arachis, Artocarpus, Asparagus, Atropa, Avena, Brassica, Carica, Citrus, Citrullus, Capsicum, Carthamus, Cocos, Coffea, Cucumis, Cucurbita, Daucus, Elaeis, Fragaria, Glycine, Gossypium, Helianthus, Heterocallis, Hordeum, Hyoseyamus, Lactuca, Linum, Lolium, Lupinus, Lycopersicon, Malus, Manihot, Majorana, Medicago, Nicotiana, Olea, Oryza, Panieum, Pannesetum, Passiflora, Persea, Phaseolus, Pistachia, Pisum, Pyrus, Prunus, Psidium, Raphanus, Ricinus, Secale, Senecio, Sinapis, Solanum, Sorghum, Theobromus, Trigonella, Triticum, Vicia, Vitis, Vigna* and Zea. The polynucleotide or vector described in accordance with the invention which is present in the multi-cellular organism may either be integrated into the genome, i.e., in the germ line of the multi-cellular organism or it may be maintained extrachromosomally, i.e., episomally.

Transgenic multi-cellular organisms that express the methylase encoding gene are expected to confer decreased resistance to specific diseases caused or enhanced by nematodes and can thus be employed in research purpose in particular in the medical field such as in the development of anti-helminthic agents. They can also be used as biological factories manufacturing the methylase, e.g., in the development of agents for treating or preventing human diseases.

Transgenic multi-cellular organisms have proved very useful in analyzing the function of gene products because a specific gene can be expressed in all the cells of a transgenic multi-cellular organism and the effect on the organism's development and function can then be monitored. This may be used in the further development of anti-helminthic agents. This is a further aspect for which the transgenic organisms can be used.

The invention also relates to a method of producing the polypeptide encoded by said nucleic acid molecule comprising growing and propagation the multi-cellular organism of the invention and isolating the produced polypeptide.

Depending on the vector constructing employed, the polypeptide may be exported to the culture medium or maintained within the multi-cellular organism. Suitable protocols for obtaining the polypeptide produced are well-known in the art for both ways of polypeptide production. For example, the polypeptide can be obtained from soya milk or mammalian milk.

For the production of the multi-cellular organism, a variety of steps are required. Thus, the nucleic acid molecule encoding the methylase must be cloned into a vector said vector containing the nucleic acid molecule may e.g. be a plasmid, cosmid, virus, bacteriophage or another vector used e.g. conventionally in genetic engineering.

The nucleic acid molecule may be inserted into several commercially available vectors. Non-limiting examples include prokaryotic plasmid vectors, such as the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Plant expression vectors comprise pGEM-T (Promega), pCAMBIA 1391 (Cambia), GATEWAY (Invitrogen), pGreen and pGreenll (PGREEN). Examples for plasmid vectors suitable for Pichia pastoris comprise e.g. the plasmids pAO815, pPIC9K and pPIC3.5K (all Intvitrogen).

The nucleic acid molecule referred to above may also be inserted into vectors such that a translational fusion with another polynucleotide is generated. The other polynucleotide may encode a protein which may e.g. increase the solubility and/or facilitate the purifcation of the fusion protein. The vectors may also contain an additional expressible polynucleotide coding for one or more chaperones to facilitate correct protein folding. For vector modification techniques, see Sambrook and Russel (2001), loc. cit. Generally, vectors can contain one or more origin of replication (ori) and inheritance systems for cloning or expression, one or more markers for selection in the host, e. g., antibiotic resistance, and one or more expression cassettes. Suitable origins of replication (ori) include, for example, the Col E1, the SV40 viral and the M 13 origins of replication.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of transcription (e. g., translation initiation codon, promoters, such as naturally-associated or heterologous promoters and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers. Preferably, the polynucleotide of the invention is operatively linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Furthermore, it is preferred that the vector comprises a selectable marker. Examples of selectable markers include neomycin, ampicillin, and hygromycine, kanamycin resistance and the like. Specifically-designed vectors allow the shuttling of DNA between different hosts, such as bacteria- fungal cells or bacteria-animal cells (e. g. the Gateway® system available at Invitrogen).

An expression vector according to this invention is capable of directing the replication, and the expression, of the polynucleotide and encoded enzyme of this invention. Suitable expression vectors which comprise the described regulatory elements are known in the art.

The nucleic acid molecules as described herein above may be designed for direct introduction or for introduction via liposomes, phage vectors or viral vectors (e.g. adenoviral, retroviral) into a cell. Additionally, baculoviral systems or systems based on Vaccinia Virus or Semliki Forest Virus can be used as eukaryotic expression systems for the nucleic acid molecules of the invention.

A typical mammalian expression vector contains the promoter element, which mediates the initiation of transcription of mRNA, the protein coding sequence, and signals required for the termination of transcription and polyadenylation of the transcript. Moreover, elements such as origin of replication, drug resistance gene, regulators (as part of an inducible promoter) may also be included. The lac promoter is a typical inducible promoter, useful for prokaryotic cells, which can be induced using the lactose analogue *isopropylthiol-b-D-galactoside*. ("IPTG"). For recombinant expression and secretion, the polynucleotide of interest may be ligated between e.g. the PeIB leader signal, which directs the recombinant protein in the periplasm and the gene III in a phagemid called pHEN4 (described in Ghahroudi et al, 1997, FEBS Letters 414:521-526). Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Alternatively, the recombinant polypeptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded polypeptide. As indicated above, the expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria. Representative examples of appropriate hosts include, but are not limited to, bacterial cells, such as *E. coli,* Streptomyces and *Salmonella typhimurium* cells; fungal cells, such as yeast cells; insect cells such as Drosophila S2 and Spodoptera Sf9 cells; animal cells such as CHO, COS, 293 and Bowes melanoma cells; and plant cells. Appropriate culture mediums and conditions for the above-described host cells are known in the art.

A method for the production of a transgenic non-human animal, for example a transgenic mouse, comprises introduction of the aforementioned nucleic acid sequence or vector into a germ cell, an embryonic cell, stem cell or an egg or a cell derived therefrom. Production of transgenic non-human embryos can be performed, e.g., as described by A. L. Joyner Ed., Gene Targeting, A Practical Approach (1993), Oxford University Press. The DNA of the embryonic membranes of embryos can be analyzed using, e.g., Southern blots with an appropriate probe; see supra. A general method for making transgenic animals is described in the art, see for example WO 94/24274. For making transgenic non-human animals (which include homologously targeted non-human animals), embryonal stem cells (ES cells) are preferred. Murine ES cells, such as AB-1 line grown on mitotically inactive SNL76/7 cell feeder layers (McMahon and Bradley, Cell 62:1073-1085 (1990)) essentially as described (Robertson, E. J. (1987) in Teratocarcinomas and Embryonic Stem Cells: A Practical Approach. E. J. Robertson, ed. (Oxford: IRL Press), p. 71-112) may be used for homologous gene targeting. Other suitable ES lines include, but are not limited to, the E14 line (Hooper et al., Nature 326:292-295 (1987)), the D3 line (Doetschman et al., J. Embryol. Exp. Morph. 87:27-45 (1985)), the CCE line (Robertson et al., Nature 323:445-448 (1986)), the AK-7 line (Zhuang et al., Cell 77:875-884 (1994)). The success of generating a mouse line from ES cells bearing a specific targeted mutation depends on the pluripotence of the ES cells (i. e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant non-human females and are born, e.g. as chimeric mice. The resultant transgenic mice are chimeric for cells having either the recombinase or reporter loci and are backcrossed and screened for the presence of the correctly targeted transgene(s) by PCR or Southern blot analysis on tail biopsy DNA of offspring so as to identify transgenic mice heterozygous for either the recombinase or reporter locus/loci.

### THE FIGURES SHOW:

**Figure 1****. Chemical structure of sterol:** R = OH, O, S, NOH; A,B,C,D = are rings in which at least one bond can be unsaturated.
**Figure 2****. Chemical structure of**: "cholesterol and cholesterone" (A), "ergosterol and ergosterone" (B) and "sitosterol and sitosterone" (C): X = OH, O, S, NOH; R = CH₃, OH, O, S, COOH, CONH₂; A,B,C,D = are rings in which at least one bond can be unsaturated.

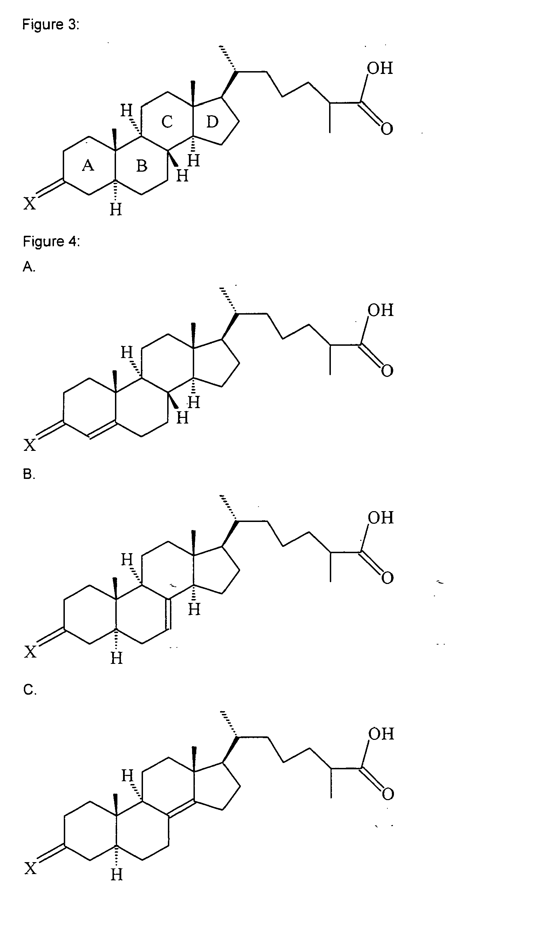

**Figure 3****. Chemical structure of dafachronic acid:** X = OH, O, S, NOH; A,B,C,D = are rings in which at least one bond can be unsaturated.

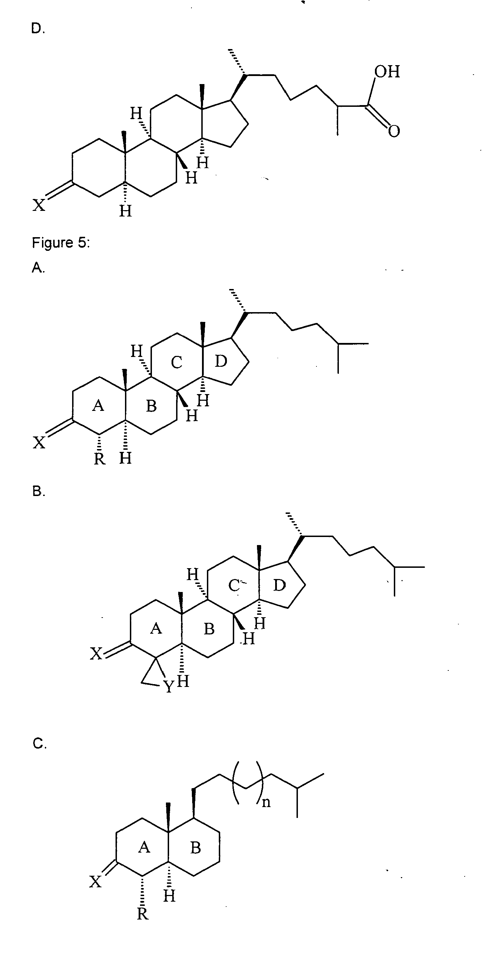

**Figure 4****. Chemical structure of:** Δ⁴-dafachronic acid (A), Δ⁷-dafachronic acid (B) Δ⁸⁽¹⁴⁾-dafachronic acid (C), or saturated dafachronic acid (D).

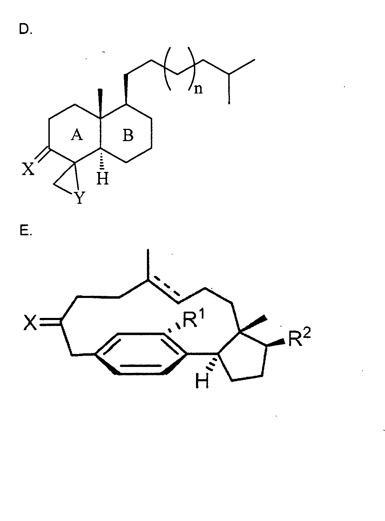

**Figure 5****. Chemical structure of a small molecules for inhibiting the function of the polypeptide as defined in the present invention:** (A,B) X = O, S, NOH; R = H, C≡CH, C≡N, N=N⁺X⁻, N₃, COOH, NO₂, Cl, Br, I; A,B,C,D = are rings in which at least one bond can be unsaturated; (C,D) n = 0 - 9; X = O, S, NOH; R = H, C≡CH, C≡N, N=N⁺X⁻, N₃, COOH, NO₂, Cl, Br, I; A,B = are rings in which at least one bond can be unsaturated; (E) X = O, S, NOH; R¹ = CHO, CH₃, CH₂OH, COOMe, COMe; R² = CH(CH₃)CH₂CH₂CH₂CH(CH₃)₂, CH(CH₃)CH=CHCH(CH₃)CH(CH₃)₂.
**Figure 6****. Effect of different sterols on dauer formation.** (A) Cholesterol in the food of wild type and *daf-12(rh61rh411)* was substituted by the depicted sterols. Red circles indicate the absence or presence of a methyl group. (B) Worms were grown for two generations and reproductive adults, dauer or arrested larvae were scored.

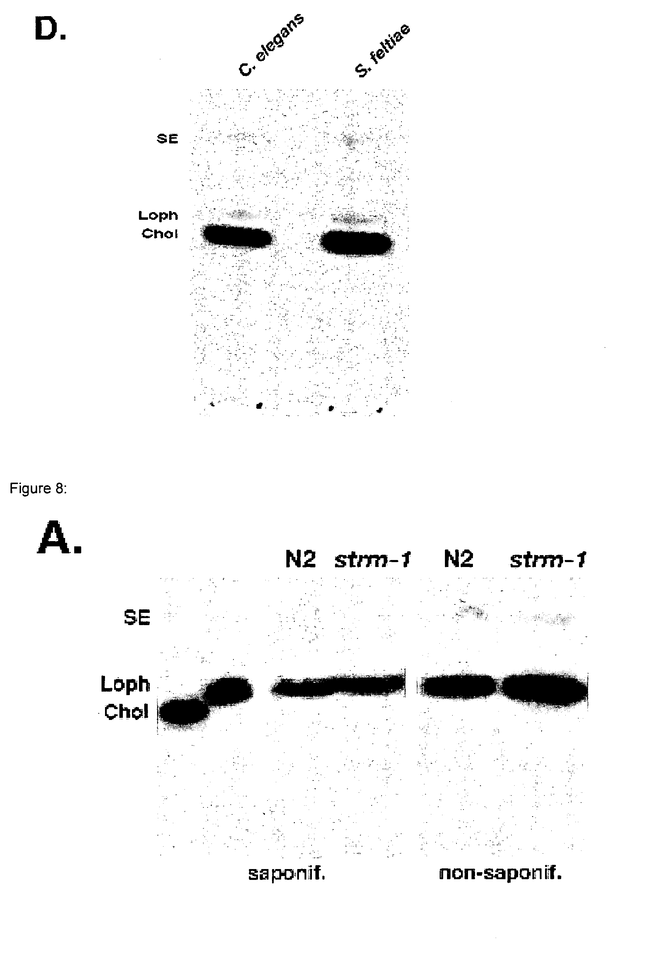

**Figure 7****. STRM-1 is responsible for C-4 methylation in the sterol nucleus**. (A) TLC of sterols from worms fed with [³H]-cholesterol showing that *strm-1(tm1781)* does not contain 4-MS. (B) TLC of labeled lipids from control RB755 (ΔR03D7.1) and ΔR03D7.1; *strm-1(tm1781)* fed with [³H]-methionine showing that STRM-1 uses S-adenosyl methionine as a co-substrate. (C) GC-MS analysis of extracts from wild type and *tm1781*(*strm-1*). Single ion modes for 7-DHC (7-dehydrocholesterol) and 4α-methyl-5α-cholest-8(14)-en-3β-ol (formula given) are shown. (D) TLC of sterols from *Caenorhabditis elegans* and *Steinernema feltiae* fed with [³H]-cholesterol showing 4-MS production in a parasitic nematode. Markers: SE - sterol esters, Loph - lophenol, Chol - cholesterol, Erg - ergosterol, CFA - 9-cyclopropylstearic acid.

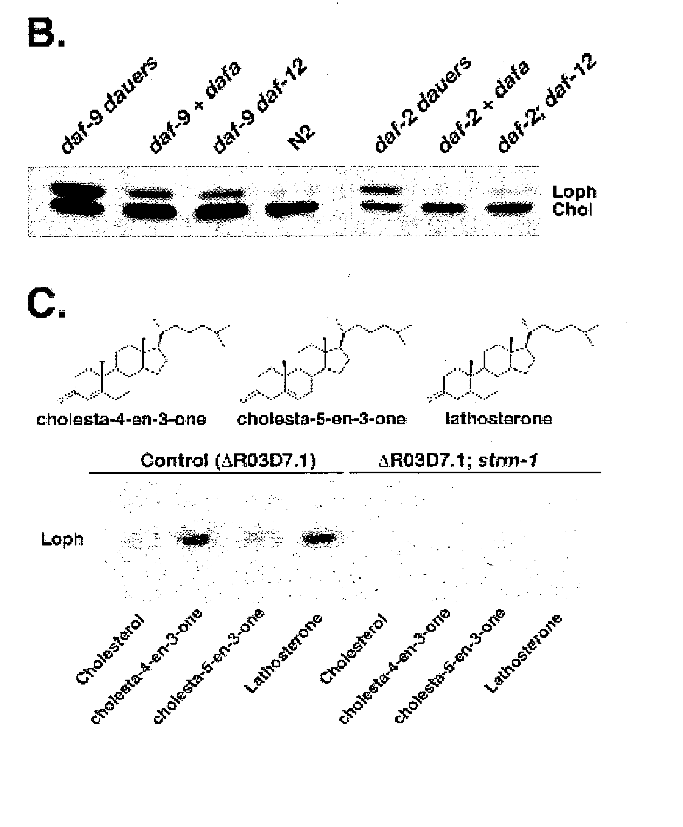

**Figure 8****. Irreversibility, regulation and substrates of cholesterol nuclear methylation.** (A) TLC-analysis of sterols after feeding with radioactive 4-MS with or without saponification. (B) TLC of ³H-cholesterol labeling in *daf-9(dh6)* and in *daf-2(e1370)* after feeding with DA or in *daf-12(rh61rh411)* background. (C) TLC of labeled 4-MS after feeding with [³H]-methionine and cold cholesterol or different ketones depicted. Markers: SE - sterol esters, Loph - lophenol, Chol - cholesterol.

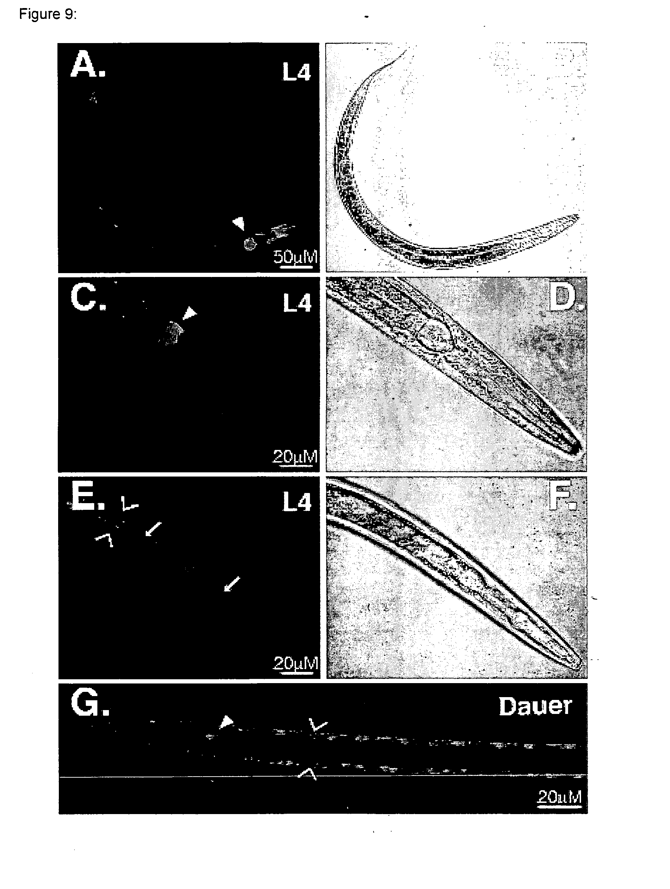

**Figure 9****. Expression pattern of STRM-1.** (A), (C) and (E) GFP fluorescence in L4 larvae from a reporter *pstrm-1::GFP.* (B), (D) and (F) Corresponding bright field images. (G) GFP fluorescence in dauer *pstrm-1::GFP; daf-7(e1372)*. Arrowheads - pharynx bulb; Arrows - seam cells; (Λ) - hypodermis.

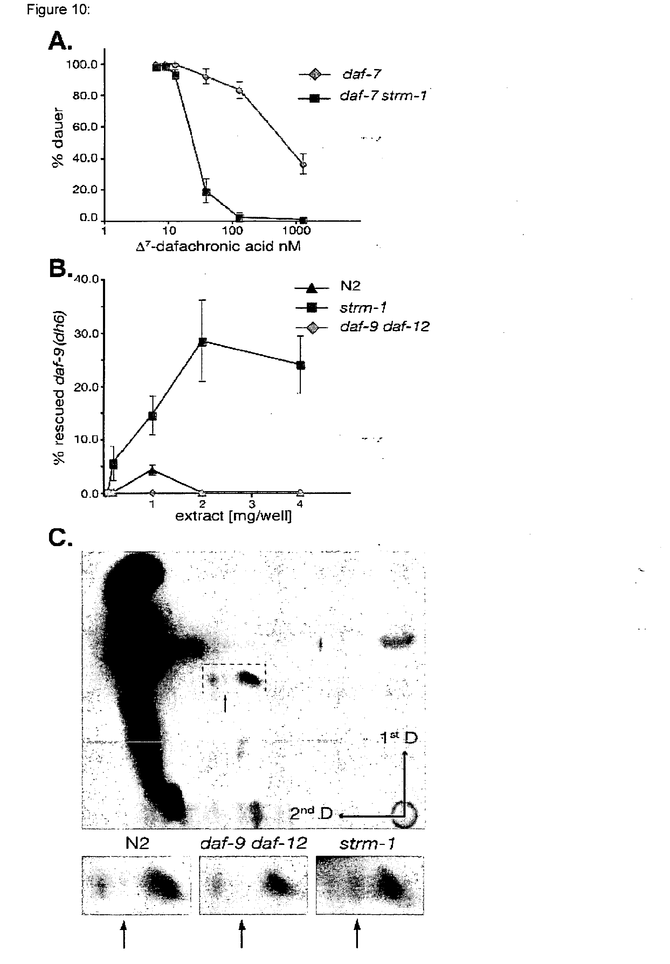

**Figure 10****. Deletion mutants of *strm-1* contain elevated amounts of DA.** (A) Bypass of dauer formation in *daf-7(e1372)* and *daf-7(e1372) strm-1(tm1781)* after feeding with DA. (B) Rescue of *daf-9(dh6)* dauer-like larvae by crude hydrophobic extracts. (C) Direct detection of elevated amounts of DAs in *strm-1(tm1781)* by 2D TLC. Upper panel - a complete TLC plate from control worms, arrows - 1^{st} and 2^{nd} dimension of the TLC. Lower panels - blowups of regions with DA, arrows - DA spot.

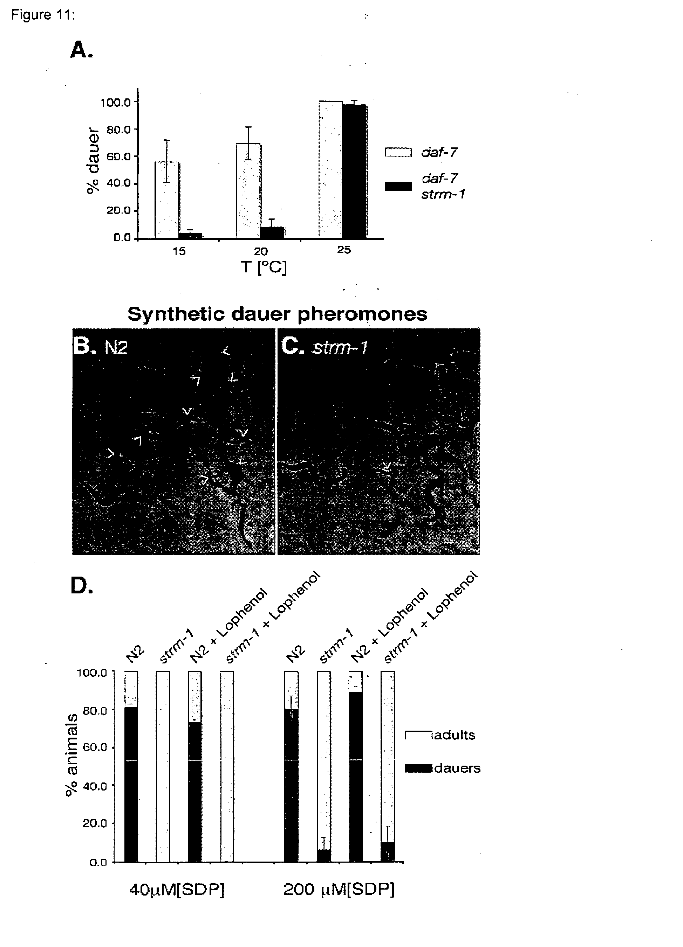

**Figure 11****. Deletion mutants of *strm-1* are inefficient in dauer larva production and do not respond to dauer inducing pheromones.** (A) Dauer formation in *daf-7(e1372)* and *daf-7(e1372) strm-1(tm1781)* at different temperatures. (B-D) Diminished response to synthetic dauer pheromones in *strm-1(tm1781).* Overview of pheromone assay plates of wild type (B) and *strm-1(tm1781)* (C). (A) indicate dauers; note, there is only a single dauer in (C). (D) Quantification of pheromone assays at different pheromone concentrations and in the presence or absence of lophenol.

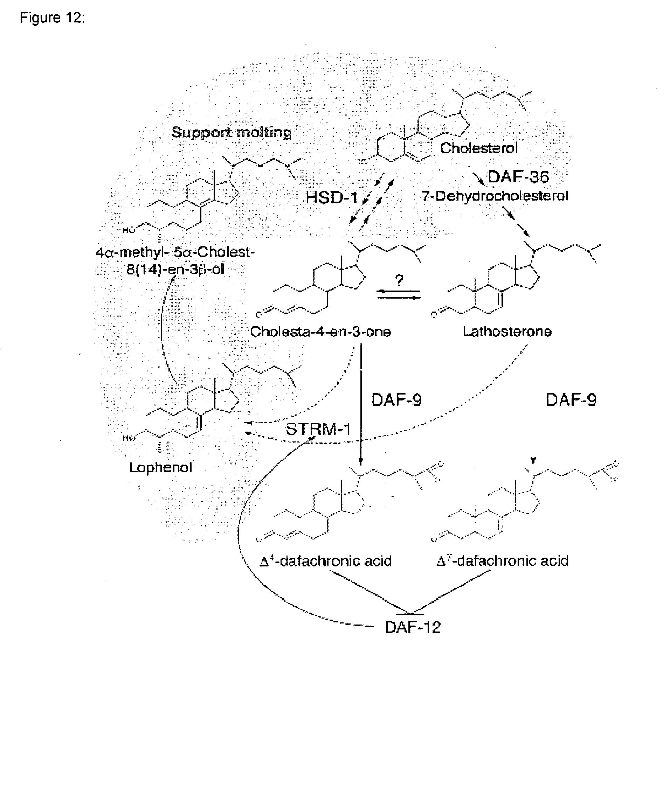

**Figure 12****. STRM-1 regulates the dauer formation process.** Cholesterol is a precursor for ketones (cholesta-4-en-3-one and lathosterone), which are substrates for DAF-9, an enzyme producing DAs. STRM-1 uses the same substrates to produce lophenol and, consequently, other 4-MS. Since the methylation of cholesterol is not reversible, 4-MS cannot be used to produce DA. Thus, STRM-1 can regulate levels of the latter and, consequently, the entry into the dauer state. Note, that in contrast to DA, 4-MS can support the molting process.

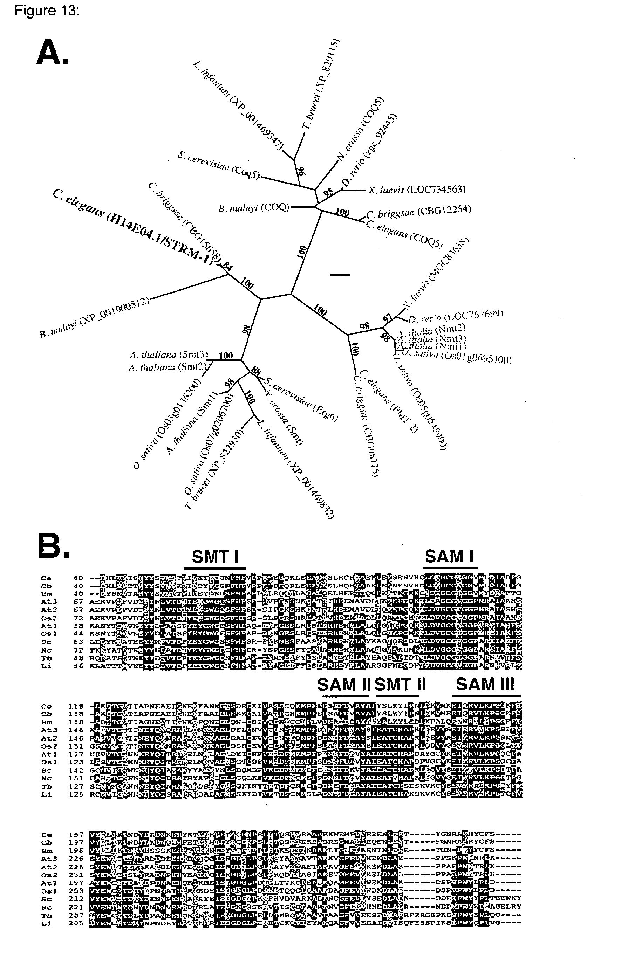

**Figure 13****. *C. elegans* genome contains a gene from the Erg6p/Sterol methyl transferase family.** (A) A phylogenetic tree of the methyltransferase 11 domain using the Erg6p/SMT family and its closest homologous families. (B) Multiple sequence alignment of H14E04.1 (STRM-1) to other members of the Erg6p/SMT family. Ce *C. elegans*, Cb *C. briggsae*, Bm *B. malayi*, At *A. thaliana*, Os *O. sativa*, Sc *S. cerevisiae*, Nc *N. crassa,* Tb *T. brucei*, Li *L. infantum*.

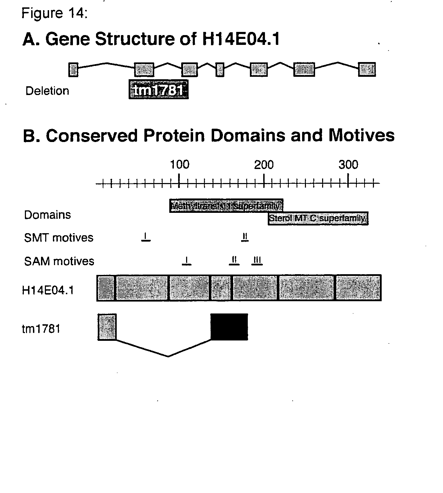

**Figure 14****. *tm1781* deletion in the gene H14E04.1.** (A) Gene structure and position of the deletion *tm1781.* (B) Conserved domains and motifs mentioned in the text. A hypothetical translation product of *tm1781* shows a frame shift resulting in a premature stop.

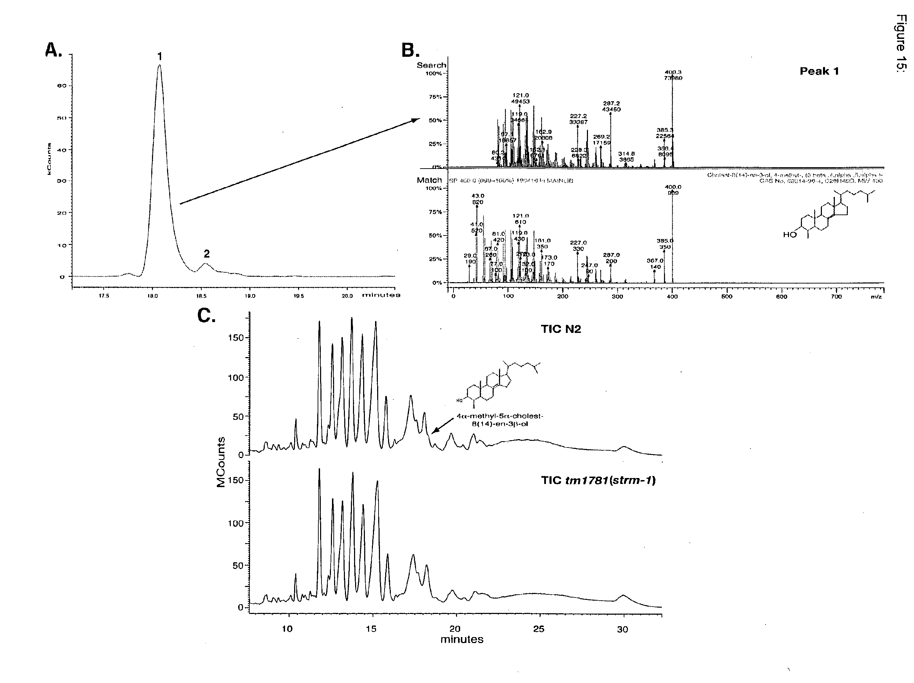

**Figure 15****. STRM-1 is responsible for the methylation of the C-4 position of the sterol nucleus.** (A) Single ion mode for m/z 400.3 of methylated sterols from *daf-9(dh6); dhEx24.* Peak 1 represents 4α-methyl-5α-cholest-8(14)-en-3β-ol, Peak 2 has the same retention time and fragmentation pattern as the lophenol standard. (B) Comparison of the fragmentation pattern of Peak 1 with a library spectrum of 4α-methyl-5α-cholest-8(14)-en-3β-ol (structure given). (C) Total ion counts of extracts from wild type (N2) and the *tm1781* mutant. Elution of 4α-methyl-5α-cholest-8(14)-en-3β-ol is indicated by an arrow.

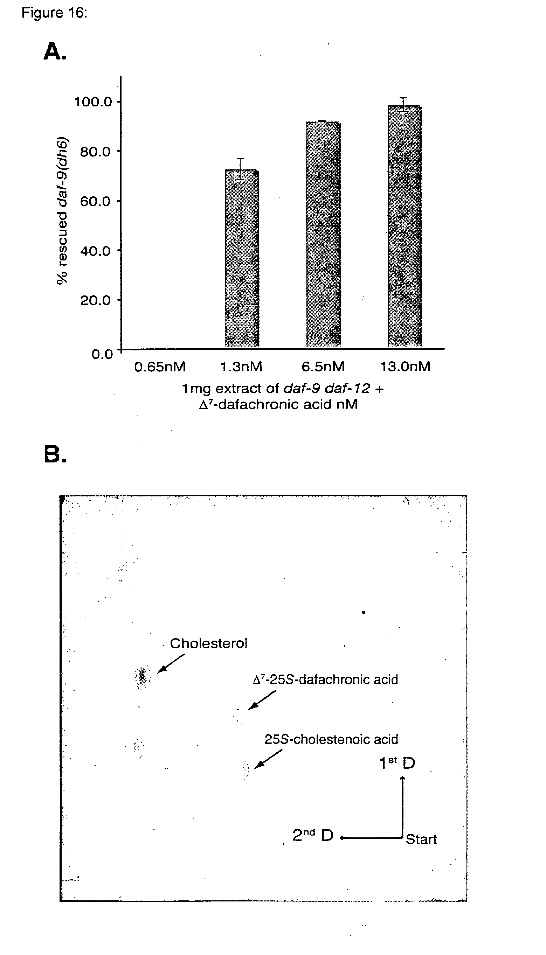

**Figure 16****. Detection of dafachronic acid levels.** (A) Bypass of dauer larva in *daf-9(dh6)* in a concentration dependent manner after supplementation of a crude hydrophobic extract from *daf-9(dh6) daf-12(rh61rh411)* with dafachronic acid. (B) Lipid standards separated in a 2-dimensional TLC corresponding to Fig. 10 C.

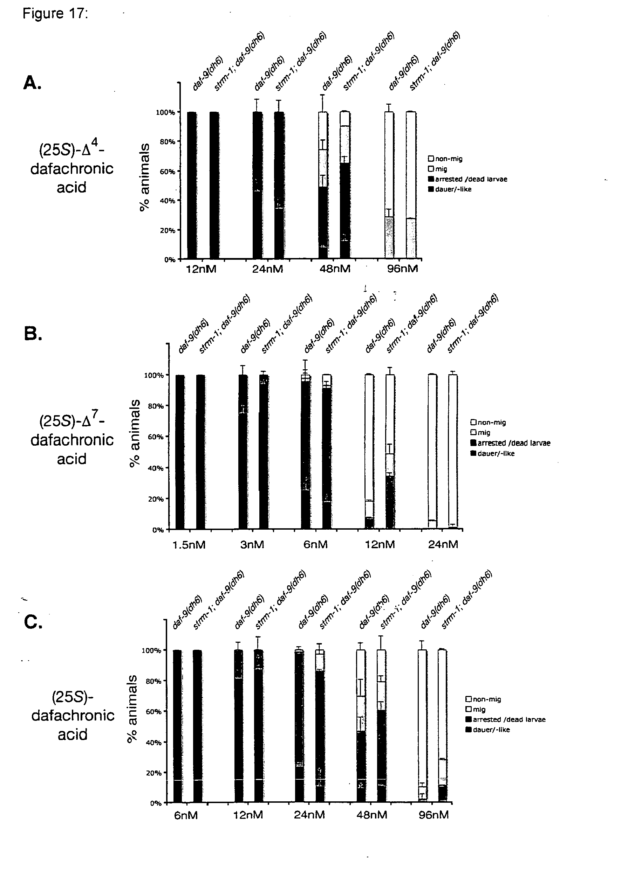

**Figure 17****. *strm-1(tm1781)* does not inactivate dafachronic acids as observed by unchanged** sensitivity to exogenously fed **dafachronic acids**. (A) (25*S*)-Δ⁴-dafachronic acid. (B) (25*S*)-Δ⁷-dafachronic acid. (C) (25*S*)-dafachronic acid. Legend (A), (B), (C): non-mig (green) -normal adults, *mig* (yellow) - animals with gonadal morphology phenotype, arrested / dead (orange) - arrested or dead larvae with molting defect or extruded gonads, dauer /-like (red) - dauer-like larvae.

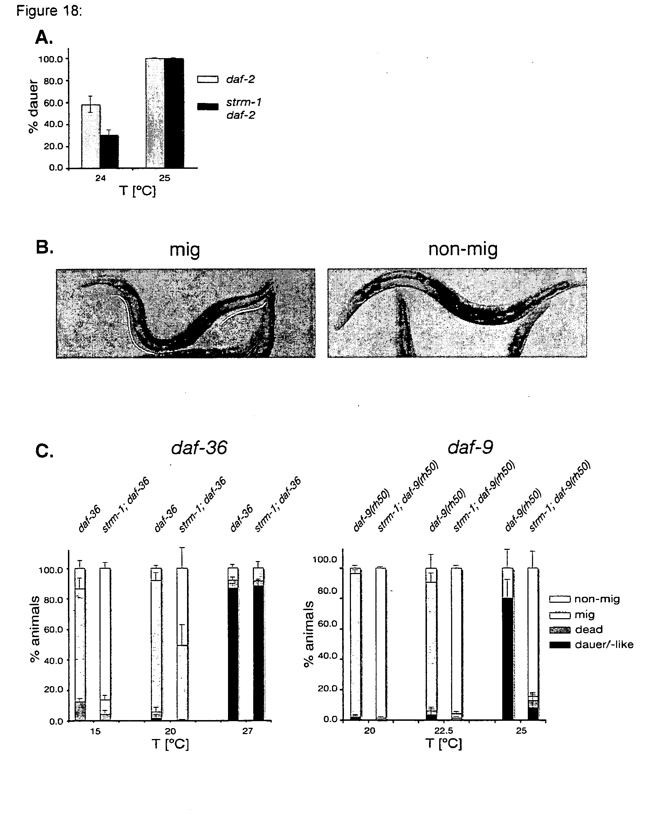

**Figure 18****. *strm-1(tm1781)* suppresses phenotypes observed when levels of dafachronic acids are reduced.** (A) Dauer formation in *daf-2(e1368)* and *strm-1(tm1781) daf-2(e1368)* at 24 and 25°C. (B) Adult worms with *mig* gonadal morphology (left panel; note arms of gonads are on the same side of the body) and non-mig WT gonad (right panel). (C) Suppression of *daf-36(k114)* and hypomorph *daf-9(rh50)* by *strm-1(tm1781)* at different temperatures. Legend: non-mig (green) -normal adults, *mig* (yellow) - animals with gonadal morphology phenotype, dead (orange) - dead animals with molting defect or extruded gonads, dauers (red) - dauer larvae.

### The examples illustrate the invention:

### EXAMPLE 1:

### Experimental procedures

### Materials

Lophenol was purchased from Research Plus (Manasquan, NJ USA), [1,2,6,7-3H]-cholesterol from Biotrends (Cologne, Germany), [methyl-³H]-methionine from GE Healthcare Europe GmbH (Freiburg, Germany), synthetic daumone from KDR Biotech Co., LTD (South Korea). Lophanol, 27-desmethylcholestanol, short ascaroside derivative of 8*R*-hydroxy-2*E*-nonenoic acid, (25*S*)-cholestenoic, (25S)- Δ⁴-, (25S)- Δ⁷- and (25*S*)-DAs were produced in the Knölker Laboratory (Martin et al. 2008, Martin et al. 2009, Martin et al., 2009, Martin et al., 2009). All other chemicals were from Sigma-Aldrich (Taufkirchen, Germany), Dulbecco's medium (DMEM) was from Invitrogen (Karlsruhe, Germany).

A list of *C. elegans* strains used in this study can be found below. *Steinernema feltiae* strain was purchased from E∼nema GmbH (Schwentinental, Germany) and grown on NGM agar plates seeded with *Xenorhabdus bovienii* at 25°C. Bacteria *Xenorhabdus bovienii* were grown in LB media at 30°C, to saturation.

### Metabolic Labeling

*Cholesterol Labeling:* about 3000 bleached embryos were put on seeded plates containing 5 to 10 µCi of [1,2,6,7-³H]-cholesterol and grown for four days until early adulthood. Large amounts of homogenous *daf-9(dh6)* dauer-like larvae were obtained by growing *daf-9(dh6); dhEx24* (see below) on NGM plates with 100nM Δ⁷-DA and isolating non-fluorescent adults (homozygous for *daf-9(dh6)*). The following generation was labeled as described above with or without DA in the medium. For S. *feltiae* labeling, adult worms were transferred to NGM plates seeded with *X. bovienii* mixed with 10 µCi [1,2,6,7-³H] cholesterol and grown for six days at 25°C.

*Methylation Labeling:* to increase label incorporation, RB755 strains were used and putative substrate sterols were added directly to the bacteria at 130 µM. For each strain and condition, about 100,000 bleached embryos were put on three 15 cm seeded plates containing 100 µCi [methyl-³H]-methionine and were grown until early adulthood.

*Lophenol Labeling:* about 40,000 *daf-7(e1372)* worms were labeled with 100 µCi [1,2,6,7-³H]-cholesterol for 2 weeks at 25°C, lipids were extracted from the resulting dauers and saponified (see below). Methylated sterols were scraped from a TLC plate, extracted from the silica powder according to (Folch et al., 1957), dried, resuspended in ethanol, mixed with sterol-free NA22 bacteria, seeded on sterol-free agarose plates and fed to worms (Matyash et al., 2004). Worms were grown for 4 days until adulthood.

### Lipid extraction and TLC analysis

Worms were lyzed by freezing/thawing and lipids were extracted according to (Bligh and Dyer, 1957) with the exception of extracts for DA analyses, which were according to (Gill et al., 2004). In cases when saponification was performed, dried lipids were heated in 3M KOH:methanol (1:9,v:v) for 1 hour at 80°C. Unsaponifiable lipids were extracted three times with hexane, pooled, dried, and used for further analysis. Cholesterol labeling samples were normalized for total radioactivity before TLC analysis; samples from the methionine labeling were normalized for total radioactivity before saponification.

Thin layer chromatography was performed on silica gel 60 or HPTLC plates (both from Merck, Darmstadt, Germany) using chloroform:methanol (24:1) as the running system. 2D TLC for the detection of DAs was done using chloroform:methanol (24:1) as 1^{st} running system and chloroform: methanol: ammonia (60:35:5) as 2^{nd}. After chromatography, plates were sprayed with scintillation fluid (Lumasafe, Lumac LSC B.V., Groningen, The Netherlands) and exposed to a film (Kodak Biomax MR, Sigma-Aldrich Chemie GmbH, Taufkirchen, Germany).

### Dauer formation assay

Worms grown at 15°C were bleached, and approx. 100 to 150 embryos were transferred to NGM 12-well plates seeded with NA22 bacteria, grown at 15, 20, 22.5, 25 or 27°C, and scored for percentage of dauer larva after three to five days depending on the temperature. In the case of *daf-36(k114)* and *daf-9(rh50)* non-mig adults, *mig* adults and larvae, dead or arrested animals with extruded gonads or molting defects, and dauer/dauer-like larvae were scored. To determine DA sensitivity in *daf-7(e1372)* and *daf-7(e1372) strm-1(tm1781),* NGM 12-well plates containing different concentrations of DA (ranging from 13 to 1300 nM) were used. To determine sensitivity of *daf-9(dh6)* and *strm-1(tm1781); daf-9(dh6)* to exogenously supplied DA, adult *daf-9(dh6); dhEx24* or *strm-1(tm1781); daf-9(dh6); dhEx24* were placed to lay about 100 eggs in 6 well plates containing different amounts of DAs. *dhEx24* is an extra-chromosomal array carrying fully active DAF-9 and also a nuclear GFP (Gerisch et al., 2001). After three days *daf-9(dh6)* were identified based on the absence of GFP fluorescence and the indicated phenotypes were scored.

### Gamravali assay and DAF-9 activity assay

Gamravali assay in Figure 11 was done according to (Matyash et al., 2004). For *daf-9* activity assay, extraction was done according to (Gill et al., 2004) and the rescue assay was performed according to (Motola et al., 2006). Approximately 500 000 bleached embryos of N2, *strm-1(tm1781)* and *daf-9(dh6) daf-12(rh61rh411)* were grown until L3 larval stage, lyzed by freezing /thawing, diluted in 15 ml TBS, mixed with 2 ml zirconium beads and extracted four times with 30 ml diethyl ether following extensive vortexing. Different dilutions of dried extracts in DMSO were mixed with plated bacteria and dried. The *daf-9(dh6)* rescue assay was performed as described above.

### Induction of dauer formation with synthetic pheromones.

The assay was done according to (Butcher et al., 2007) but two synthetic dauer inducing pheromones (daumone (Jeong et al., 2005) and short ascaroside derivative of 8*R*-hydroxy-2*E*-nonenoic acid (Butcher et al., 2007)) were used in combination at relatively high concentrations to ensure a strong and robust response in wild type (see Fig. 11). Pheromones were mixed both to NGM and to heat inactivated bacteria. In the indicated cases lophenol (13 µM) was added to the seeded bacteria. Adult worms were placed to lay eggs (approx. 150 per well in 6 well plates). After 2 days at 25°C, dauers and L4 larvae, or young adults were counted.

### Nematode strains

The following strains were used: N2 (Bristol strain), RB755 R03D7.1*(ok521)*, *daf-7(e1372), strm-1(tm1781)* (6x outcrossed), *daf-2(e1368), daf-2(e1370), daf-36(k114), daf-9(rh50)*, *daf-9(dh6); dhEx24* (containing the cosmid T13C5 and pTG96 [*sur5::gfp*]), R03D7.1*(ok521); strm-1(tm1781), daf-7(e1372) strm-1(tm1781), strm-1(tm1781) daf-2(e1368), strm-1(tm1781) daf-2(e1370), strm-1(tm1781); daf-36(k114), strm-1(tm1781); daf-9(rh50), strm-1(tm1781); daf-9(dh6); dhEx24, daf-9(dh6) daf-12(rh61rh411). strm-1(tm1781)* was received from Dr. S. Mitani at NBRP, *daf-9(dh6) daf-12(rh61rh411)* was from Dr. A. Antebi, all other single mutants from the Caenorhabditis Genetics Centre. *C. elegans* strains were routinely propagated on NGM-agar plates seeded with NA22 bacteria at 20°C as previously described (Brenner, 1974), unless indicated otherwise.

### Cloning of pstrm-1::GFP

The perspective *strm-1* promoter region was PCR amplified from genomic DNA (primers atttttcaactaaaatcatta and atgtttatagacgaagaaattgg) and cloned via SacII and BamHI into the TH304-GFPc-Amp vector (Hyman Lab, MPI-CBG, Dresden). Several transgenic worm lines were obtained by ballistic transformation of *unc-119(ed-3)* (Wilm et al., 1999). Light and fluorescence microscopy was done according to (Matyash et al., 2004).

### Cloning of ADH1::STRM-1

For generation of the yeast expression construct, H14E04.1 cDNA was PCR amplified from the yk401g2 phage clone (from the National Institute of Genetics, Mishima, Japan; primers cgggatccatgtccatcaatatgaatgcc and ggctcgagtcagattttcttcttctcaaa with added BamHI and Xhol restriction enzyme recognition sequences, respectively). The cDNA was first cloned into the pEG(KT) vector via BamHI and SalI and later subcloned via BamHI and SacI into the integrative -URA vector YIplac211 (Gietz and Sugino, 1988) containing the S. *cerevisiae* ADH1 promoter (1.5kb HindIII BamHI fragment from pAAH5, (Ammerer, 1983)). Constructs were integrated into the URA promoter region of Euroscarf WT (BY4741) and the corresponding *erg6*Δ (YML008c::kanMX4) strains following Nsil linearization.

### Sequence and phylogenetic analysis

For the alignment of sterol methyltransferases the following sequences were used: Ce *C. elegans* NP 497549.2, Cb *C. briggsae* XP 001664373.1, Bm *B. malayi* XP 001900512.1, At1 *A. thaliana* NP 196875.1, At2 A. *thaliana* NP 173458.1, At3 A. *thaliana* NP 177736.1, Os1 *O. sativa* NP 001048891.1, Os2 *O. sativa* NP 001059154.1, Nc *N. crassa* XP 965392.1, Sc *S. cerevisiae* NP 013706.1, Tb *T. brucei* XP 822930.1, Li *L. infantum* XP 001469832.1. Sequences were aligned using ClustalW, conserved residues were visualized using boxshade. For the larger phylogenitic tree additional sequences were added: *C. briggsae* CBG08775 XP 001672563.1, *C. elegans pmt-2* NP 504248.1, *C. briggsae* CBG02363 XP 001679815.1, *C. elegans pmt-1* NP 494991.1, *O. sativa* Os05g0548900 NP 001056231.1, A. *thaliana* NMT2 NP 973993.1, A. *thaliana* NMT1 NP 188427.2, *O. sativa* Os01g0695100 NP 001043957.1, A. *thaliana* NMT3 NP 177501.1, *D. rerio* LOC767699 NP 001070105.1, *X. laevis* MGC83638 NP 001087172.1, *C. elegans* coq-5 NP 498704.1, *C. briggsae* CBG12254 XP 001666262.1, *B. malayi* ZK652.9 XP 001896724.1, *S. cerevisiae* Coq5p NP 013597.1, *N. crassa* coq5 XP 960179.1, *X. laevis* LOC734563 NP 001089511.1, *D. rerio* zgc:92445 NP 001004541.1, *G. gallus* COQ5 NP 001006194.1, *M. musculus* COQ5 NP 080780.1, *H. sapiens* COQ5 NP 115690.3. Only the methyltransferase 11 domain (pfam08241) was used for a ClustalW multiple sequence alignment. For proteins with two methyltransferase domains, only the C-terminal domain was used. The tree was estimated by the maximum likelihood approach implemented in the phyML package using the JTT model of protein evolution with gamma distributed rates across sites.

### Sterol analysis by mass spectrometry

For 4-MS determination, approx. 40,000 young adults were harvested, lysed and extracted as described in Experimental Procedures. Lipid extracts were saponified, and the unsaponified lipids were extracted with hexane, fatty acids were removed using a QAE-Sephadex A-50 column (Pharmacia Fine Chemicals AB, Uppsala, Sweden) and the remaining lipids were analyzed by GC/MS. Samples were dissolved in chloroform/methanol (2:1 v/v) and injected into a VARIAN CP-3800 Gas Chromatograph equipped with a Factor Four Capillary Column VF-5ms (30m x 0.25mm ID DF=0.25) and analysed by a Varian 320 MS triple quadrupole with electron energy set to -70 eV at 200°C and the transfer line at 280°C. The temperature was held for 4 min at 45°C, ramped successively to 195°C (20°C/min), to 230°C (4°C/min), to 325°C (20°C/min), to 350°C (6°C/min) before cooling back to 45°C. Free sterols were eluted during the linear gradient from 195-230°C. 7-dehydrocholesterol (m/z=384.3) and 4α-methyl-5α-cholest-8(14)-en-3β-ol (m/z=400.3) were identified by comparison of elution profiles and fragmentation patterns with reference to the NIST database and standards. Relative quantities of the two sterols were determined by extraction of the 384.3 and 400.3 ions.

### EXAMPLE 2:

### Effects of sterol A-ring and side chain methyl groups on dauer larva formation

In *C. elegans,* the substitution of dietary cholesterol with 4-MS after one generation leads to dauer formation (Matyash et al., 2004). 4-MS can neither bind to DAF-12 to inhibit dauer formation nor can they function as precursors for the reproduction hormone DA. However, 4-MS are sufficient for molting, another important function of sterols in nematodes (Entchev and Kurzchalia, 2005; Kurzchalia and Ward, 2003) as a null mutant of *daf-12* can grow and molt normally when supplied only with 4-MS (Matyash et al., 2004). Partially a substance was purified that rescues the effect of 4-MS and called it gamravali (Matyash et al., 2004). Recently, Motola et al. identified Δ⁴- and Δ⁷-DAs as products of DAF-9 and ligands of the nuclear hormone receptor DAF-12 (Motola et al., 2006) (Fig. 6A). DAs were synthesized (Martin et al., 2008) and tested whether they abolish the 4-MS effect. It was found that worms grown on a mixture of 4-MS lophanol and DA enter the reproductive cycle (Fig. 6B; Only Δ⁷-DA is indicated). Like gamravali, DA alone cannot substitute for cholesterol and worms are arrested as L2 larvae (Fig. 6B; (Matyash et al., 2004)). Thus, DA fulfils the activity definition of gamravali and could potentially be identical to it.

The 25*S*-DAs are 3-keto-derivatives of the 25*S*-cholestenoic acid and DAF-9 is the enzyme proposed to hydroxylate the C-26 position. The role of the branch point of the side chain (C-25 position) in dauer formation was tested. 27-desmethylcholestanol (Fig. 6A; (Martin et al., 2009)) was synthesized and fed to worms as the sole source of sterols. 27-desmethylcholestanol led to dauer formation in the second generation (Fig. 6B). Upon addition of DA, however, worms entered the reproductive cycle. Therefore, to produce a functional DA, an intact branch in the side chain is required. This suggests that either DAF-9 needs the branch for its activity or DAF-12 can bind only an acid that has this branch. Interestingly, the branch is not needed for the structural organization of the membrane, since *daf-12(rh61rh411)* mutants that cannot produce dauers grow and reproduce on 27-desmethylcholestanol (Fig. 6B).

Thus, methyl groups at the C-4 and C-25 positions have opposite effects on dauer production: the presence of the former induces dauers, whereas the presence of the latter is needed for reproduction. In contrast to the C-25 methyl group missing in the artificial 27-desmethyl compound, worms can actively incorporate a missing methyl group into the C-4 position. Therefore, it was to determine whether this modification could be involved in the regulation of dauer formation and the corresponding methylase could be part of the dauer-producing pathway.

### EXAMPLE 3:

### Identification of a novel methylase responsible for the methylation of the sterol nucleus at the C-4 position

So far, nuclear methylation of sterols in the A-ring has been described only in nematodes (Chitwood, 1999; Chitwood et al., 1983). It is known, however, that in budding yeast Erg6p methylates sterols in the C-24 position, utilizing a Δ²⁴ precursor, zymosterol (Gaber et al., 1989). Similar enzymes, called SMTs (Sterol Methyl Transferases) have also been described in plants (Grebenok et al., 1997), *Trypanosoma* (Zhou et al., 2006) and *Leishmania* (Magaraci et al., 2003). The putative 4-methylase of nematodes could belong to the Erg6p/SMT family and contain the common methyltransferase domain (pfam08241, methyltransferase 11) as well as the specific SMT C-terminal domain (pfam08498). A phylogenetic tree of the methyltransferase 11 domain, using the SMT family and its closest homologous families, is shown in Fig. 18A. The *C. elegans* genome encodes only for one gene product that clusters with Erg6p: H14E04.1 (see Fig. 14). A multiple sequence alignment with other members of the *Erg6p*/SMT family is given in Fig. 13B. The S-adenosyl methionine (SAM)-binding motifs (Kagan and Clarke, 1994) are highly conserved in H14E04.1. The two SMT motifs (Grebenok et al., 1997) that are important for sterol binding in yeast Erg6p (Nes et al., 2004) are less conserved in H14E04.1, indicating a different architecture of the substrate pocket, which would allow for methylation at a different location on the sterol. Interestingly, the new diverged subfamily of SMT enzymes includes members in both free living (*C. elegans* and *C. briggsae*) and parasitic nematodes (*B. malayi*; Fig. 13A).

To test whether H14E04.1 is responsible for the production of 4-MS a deletion mutant was used affecting this locus. In the allele *tm1781,* two exons encoding conserved protein motifs are missing (Fig. 14), and potential splicing would lead to a frame shift that generates an early stop codon. Thus, the allele *tm1781* should be a genetic null. The mutant was fed with radioactive cholesterol and its sterols were analyzed by TLC. As seen in Fig. 7A, the deletion mutant of H14E04.1 displays no band with an R_{f} identical to that of 4-MS.

H14E04.1 has a readily discernible SAM-binding domain and thus should use SAM as a co-substrate in the methylation reaction. It was tested whether radioactive methionine could label 4-MS (Fig. 7B). To increase the label incorporation, the deletion mutant *ok521* of methionine synthase R03D7.1 was exploited, with a deficiency in methionine biosynthesis (J.T.H. and T.V.K., unpublished). Due to the deletion in R03D7.1, the RB755 worm strain relies on exogenous methionine, supplied by its bacterial food source. The major band of labeled lipids corresponds to cyclopropyl fatty acids (CFA). A band with R_{f} identical to 4-MS present in wild type animals is entirely absent when H14E04.1 is deleted. Note that *C. elegans* appears not to produce 24-methyl sterols (ergosterols), as a corresponding band was not detected.

The absence of 4-MS in *tm1781* was confirmed by GC-MS analysis (Fig. 7C). Different nuclear methylated sterols could also be distinguished (Iophenol or 4α-methyl-5α-cholest-8(14)-en-3β-ol; (Chitwood et al., 1983)). In sterols from a mixed population of worms, two peaks with a mass-to-charge ratio (m/z) of 400.3 (Fig. 15A) were detected. The minor peak 2 has the same retention time and fragmentation spectrum as the lophenol standard, while the major peak elutes earlier and matches to the library spectrum of 4α-methyl-5α-cholest-8(14)-en-3β-ol (Fig. 15B). After saponification of total lipid extracts from young adults of wild type and *tm1781,* GC-MS spectra show very little over-all differences in the total ion counts (Fig. 15C). The deletion mutant of H14E04.1 has comparable to wild type amounts of desmethylsterols (shown for 7-dehydrocholesterol in Fig. 7C). In *tm1781,* the peaks of 4-MS are not detected (Fig. 7C) indicating that H14E04.1 is needed for their production.

To address whether H14E04.1 is specific for sterol methylation at the C-4 position, it was tested whether H14E04.1 can complement *erg6*Δ in *S. cerevisiae.* Erg6p is a sterol methylase in budding yeast that methylates the side chain of zymosterol at C-24, producing fecosterol further metabolized to ergosterol (Gaber et al., 1989). H14E04.1 was cloned and expressed in yeast *erg6*Δ. It was found that H14E04.1 cannot produce ergosterol to restore normal growth of the *erg6*Δ (data not shown). Therefore, H14E04.1 appears to be specific for sterol C-4 methylation.

Taken together, the structural features of H14E04.1 (presence of SAM and SMT domains), the absence of 4-MS in *tm1781,* and the inability to complement *erg6*Δ indicate that H14E04.1 methylates sterols in position C-4 and is therefore the STerol A-Ring Methylase (STRM-1).

It was investigated whether methylation of sterols in the ring is indeed conserved in nematodes. For this purpose, a culture of an insect parasitic nematode *Steinernema feltiae* was labeled with radioactive cholesterol. *C. elegans* and *S. feltiae* belong to different nematode suborders (Nadler et al., 2006). However, as seen in Fig. 7D, the patterns of sterols in *C. elegans* and *S. feltiae* are very similar: in both species, 4-MS are major constituents.

### EXAMPLE 4:

### Nuclear methylation of cholesterol is irreversible

Organisms with *de novo* biosynthesis of sterols have an elaborate system for demethylation of lanosterol or cycloartenol (methylated precursors of cholesterol and ergosterol or sitosterol, respectively) at the C-4 position (Benveniste, 2004; Gaylor, 2002)). In contrast, nematodes actively methylate this position. Is the A-ring methylation reversible in *C. elegans*?

To investigate this question, radioactive 4-MS was prepared from worms labeled with ³H-cholesterol (see Experimental Procedures). When fed to wild type and *strm-1(tm1781)* worms, radioactive 4-MS were taken up and accumulated to significant levels (Fig. 8A). These 4-MS were actively metabolized as the label was found also in sterol esters (Fig. 8A, non-saponified sample). However, even after several months of exposure any traces of cholesterol/desmethylsterol in either the original extracts or in saponified samples could be detected (although, a very faint hydrophilic band appeared close to the start, not shown). The possibility that cholesterol derived from Iophenol is quickly re-methylated could be excluded, as *strm-1(tm1781)* also did not show any cholesterol signal (Fig. 8A). Thus, methylation of the cholesterol nucleus in nematodes appears to be irreversible.

### EXAMPLE 5:

### Methylated sterols are strongly elevated in dauer larvae and STRM-1 acts downstream of DAF-9 and DAF-12

To investigate whether there is a direct connection between dauer formation and sterol methylation, the levels of 4-MS in DAF-9-deficient animals were examined (Fig. 8B) by analyzing the pattern of sterols in *daf-9(dh6)* dauer-like larvae. A large homogenous population of *daf-9(dh6)* dauer-like larvae was obtained by feeding the previous generation with DA (see Experimental Procedures). In *daf-9* deficient dauer-like larvae 4-MS are highly increased in comparison to the reproductive stages (Fig. 8B). If *daf-9(dh6)* are fed with DA, 4-MS are reduced but still elevated in comparison to the wild type. Densitometric analysis of radiographs showed that when exogenous DA is added to *daf-9* mutants, the ratio of 4-MS/nMS is about two to three times higher than in the wild type. This elevation can also be seen in a *daf-9(dh6) daf-12(rh61rh411)* double mutant (Fig. 8 B). In this strain, the receptor for DA, DAF-12, is inactive and mutants grow normally despite lacking DAF-9 and, consequently, DA. Thus, the absence of DA synthesis elevates the level of methylated sterols present.

To evaluate the significance of *daf-12* in regulating 4-MS levels in dauers an experiment was designed in which dauer-like larvae with inactivate *daf-12* were obtained. To this end radioactive cholesterol was fed to a strong *daf-2* allele, *e1370*, which cannot completely exit the dauer stage even by feeding with DA or in a *daf-12* null background, and in both conditions such animals remain as arrested larvae (Gems et al., 1998; Motola et al., 2006). Control *daf-2(e1370)* dauers showed a similar ratio of 4-MS/nMS to that seen in *daf-9(dh6)* dauer-like larvae (Fig. 8B). Interestingly, in *daf-2(e1370)* animals fed with DA, or in a *daf-12(rh61rh411)* background, 4-MS were reduced to a ratio similar to *daf-9(dh6)* fed with DA and *daf-9(dh6) daf-12(rh61rh411)*. Taken together, these results indicate that: i) 4-MS strongly accumulate in dauers when there is no *daf-9* activity; ii) during reproductive development, if *daf-9* is missing, there is still elevation of 4-MS; iii) accumulation of 4-MS during dauer formation is *daf-12* dependent, as 4-MS are reduced in dauer-like *daf-2(e1370)* larvae obtained either with DA or depletion of *daf-12*. Thus, the activation of the dauer program via DAF-12 in the absence of DA leads to an increase in sterol methylation.

It was hypothesized that nematodes may exploit methylation of the A-ring in sterols in order to regulate the amount of DA. One possibility is that the enzyme producing 4-MS competes with DAF-9 for a substrate(s). Δ⁴- and Δ⁷-DAs are produced from the corresponding cholesten-3-ones ((Motola et al., 2006); formulae in Fig. 8C). To test whether these cholesten-3-ones were used as substrates for the production of 4-MS, wild type and *strm-1(tm1781)* worms were fed with cholesten-3-ones and the methylation was followed by radioactive methionine, as described above (Fig. 8C). It was found that cholest-4-en-3-one and cholest-7-en-3-one (lathosterone), also substrates of DAF-9, are the most effective precursors for the production of 4-MS.

### EXAMPLE 6:

### The Expression patterns of STRM-1 and DAF-9 partially overlap

Competition between enzymes for a substrate is possible if they are expressed in the same cells. *daf-9* is expressed in the XXX cells, the hypodermis, and the spermatheca (Gerisch and Antebi, 2004; Gerisch et al., 2001; Jia et al., 2002; Mak and Ruvkun, 2004). The expression pattern of *strm-1* using a GFP reporter was investigated. *strm-1* expression starts late in embryogenesis and it is high throughout larval stages and in adults (not shown and Fig. 9A, B). During reproductive development, the strongest expression was detected in the pharynx (Fig. 9C, D), which is one of the major sites of sterol accumulation (Matyash et al., 2001). The hypodermal syncytium also showed high levels of fluorescence (Fig. 9E, F) in contrast to the seam cells (white arrows). In order to follow *strm-1* expression in dauer larvae, the reporter in *daf-7(e1372)* animals was introduced which produce dauers at 25°C (Fig. 9G). While pharyngeal fluorescence in dauers decreases significantly, GFP in the hypodermis remains at high levels indicating high expression of STRM-1 in dauers.

Previously, it was shown that *daf-9* is expressed in two neuron-like XXX cells that are important in preventing dauer arrest (Gerisch et al., 2001; Jia et al., 2002). *daf-9* is also strongly expressed in the hypodermis during reproductive growth, but shut down in dauers (Gerisch and Antebi, 2004; Mak and Ruvkun, 2004). Thus, *strm-1* and *daf-9* are present in the same hypodermal cells and would be able to compete for substrates (cholesten-3-ones) during reproductive development. In addition, *strm-1* continues to be expressed in dauers, which accumulate 4-MS ((Matyash et al., 2004) and Fig. 8B) and, presumably, do not produce DA.

### EXAMPLE 7:

### Deletion mutants of strm-1 contain higher amounts of DA

It was documented above that the absence of DAF-9 can elevate 4-MS. A related question is whether deletion of *strm-1* elevates DA and thus makes the production of dauer larvae more difficult. To investigate the influence of *strm-1* on dauer formation, double mutants of *strm-1(tm1781)* and *daf-*7(e1372) were produced which at 25°C produces dauers. *daf-7* mutants can be rescued by feeding with DA (Motola et al., 2006). If *daf-7(e1372) strm-1(tm1781)* have elevated DA then worms grown on different concentrations of hormone should be rescued to a greater extent than *strm-1* positive animals. At high concentrations, DA rescues dauer formation in both *daf-7(e1372)* and in *daf-7(e1372) strm-1(tm1781)* (Fig. 10A). The differences become evident at lower concentrations: ten times less DA still can fully rescue the double mutant. Remarkably, the addition of lophenol/lophanol to the double mutants does not decrease their sensitivity to DA (not shown). This again indicates that 4-MS do not actively induce dauer formation but instead suggest that their biosynthesis influences amounts of the hormone by competing with substrates of DAF-9 (see also the effect of lophenol later).

Next it was decided to the compare level of DA in wild type and *strm-1(tm1781)* animals based on its bio-activity, using the rescue of *daf-9(dh6)* mutants as an assay (Gerisch et al., 2007; Motola et al., 2006). DA rescues DAF-9 deficiency in a concentration dependent manner (Motola et al., 2006). The activity of extracts from wild type, *strm-1(tm1781)* and *daf-9(dh6) daf-12(rh61rh411)* worms was tested (Fig. 10B). As expected, the extract from *daf-9(dh6) daf-12(rh61rh411)* was inactive (note that addition of DA to the extract rescued *daf-9* deficient animals (Fig. 16A)). In contrast, crude extracts from wild type and *strm-1(tm1781)* rescued *daf-9* deficiency, with the *strm-1(tm1781)* extract being more active. It could be diluted five- to ten-fold and still had a comparable activity to wild type extracts.

The higher activity of the extract from *strm-1(tm1781)* could be based on the ability of STRM-1 to methylate DA leading to inactivation of the hormone. In order to investigate this, we exploited the sensitivity of *daf-9(dh6) mutants* to exogenously supplied DAs in a *strm-1(tm1781)* background was ex. It was reasoned that if STRM-1 is inactivating DA, the double mutant *strm-1(tm1781); daf-9(dh6)* would be more sensitive to exogenously supplied hormone than the single *daf-9(dh6)* mutant. This could be detected either by rescue at low concentrations that have no effect on *dh6* alone, or by increased rescue at suboptimal concentrations. As seen in Fig. 17, *strm-1(tm1781)* does not lead to increased sensitivity to exogenous DAs in this *daf-9* null background since both lines need similar amounts of DAs to enter into reproduction. Thus, direct inactivation of the hormone by methylation seems very improbable. Obviously, the differences in amounts of DA between wild type N2 and *strm-1(1781)* should be demonstrated directly. To detect DA(s) on TLC, a 2D-system was designed where acidic sterols were clearly separated from non-charged ones. (Separation of DA, cholestenoic acid and cholesterol is shown in Fig. 16B). In an extract from control N2 worms, three cholesterol-derived spots with acidic properties appeared in the region where the DA standard is located (Fig. 10C, rectangle). The right and left spots are present in *daf-9 daf-12* (lower panel) as well as in dauers (not shown). The middle one, however, with identical R_{f}-values to the DA standard, is missing in the extracts from *daf-9 daf-12* (lower panel) or dauers. It is concluded that this spot is DA. Remarkably, the intensity of the spot increases significantly in the extract from *strm-1(tm1781)*. This spot is very faint even in N2, which explains the low activity of this extract (Fig. 10B). All attempts to detect this difference by LC-MS and thus quantify the increase of DA in *strm-1(tm1781)* have been unsuccessful. Thus, it seems that the absence of STRM-1 leads to an elevated level of DA, and consequently this methylase may well regulate dauer formation.

### EXAMPLE 8:

### Deletion mutants of strm-1 are inefficient in dauer larva production

The efficiency of dauer formation of *daf-7(e1372)* and *daf-7(e1372) strm-1(tm1781).* at different temperatures was compared (Fig. 11A). Although both mutant strains produce dauers at 25°C, at lower temperatures the number of reproductive animals in the double mutant was much higher (Fig. 11A). The *daf-7(e1372)* mutant produces dauers even at the permissive temperature (15°C) ((Swanson and Riddle, 1981) and Fig. 11A; about 50% at 15°C). In contrast to this, only about 3% dauer formation was observed in the *daf-7(e1372) strm-1(tm1781)* double mutant.

The influence of STRM-1 on the efficiency of dauer formation was tested also in other genetic backgrounds *(daf-2(e1368), daf-36(k114)* and *daf-9(rh50)*) (Fig. 18). In all cases, a very similar tendency to that seen in *daf-7* mutants was observed. Remarkably, *strm-1(tm1781)* was very effective in rescuing the distal tip cell migration defect (Fig. 18B), a characteristic of some daf-c hypomorphs, which develops at low levels of hormone (Gerisch et al., 2001; Jia et al., 2002; Rottiers et al., 2006). Thus, hypomorphic *daf-9(rh50)* mutants could be rescued almost entirely by *strm-1(tm1781).* These data argue that there is a genetic interaction between *daf-9* and *strm-1.*

The most direct proof that STRM-1 regulates the process of dauer formation under natural conditions was obtained using dauer-inducing pheromones (daumone/short ascarosides). These substances induce dauers effectively in wild type worms (Fig. 11B,D). In contrast, the ability of *strm-1(tm1781)* mutants to produce dauers was dramatically impaired and the inducing pheromones were needed in at least an order of magnitude higher amounts. The addition of lophenol had no effect on the production of dauers in the absence or presence of pheromone. As the amount of pheromone is directly proportional to the number of worms, animals lacking *strm-1* will produce dauers only under very crowded conditions. This makes the process very uncommon/unlikely and again demonstrates that STRM-1 activity is a major component in the regulation of dauer formation.

### REFERENCES

Ammerer, G. (1983). Expression of genes in yeast using the ADCI promoter. Methods Enzymol 101, 192-201.
Antebi, A., Culotti, J.G., and Hedgecock, E.M. (1998). daf-12 regulates developmental age and the dauer alternative in Caenorhabditis elegans. Development 125, 1191-1205.
Antebi, A., Yeh, W.H., Tait, D., Hedgecock, E.M., and Riddle, D.L. (2000). daf-12 encodes a nuclear receptor that regulates the dauer diapause and developmental age in C. elegans. Genes Dev 14, 1512-1527.
Benveniste, P. (2004). Biosynthesis and accumulation of sterols. Annu Rev Plant Biol 55, 429-457. Bligh, E.G., and Dyer, W.J. (1957). A rapid method of total lipid extraction and purification. Can J Biochem Physiol 37, 911-917.
Brenner, S. (1974). The genetics of Caenorhabditis elegans. Genetics 77, 71-94.
Butcher, R.A., Fujita, M., Schroeder, F.C., and Clardy, J. (2007). Small-molecule pheromones that control dauer development in Caenorhabditis elegans. Nat Chem Biol 3, 420-422.
Chitwood, D.J. (1999). Biochemistry and function of nematode steroids. Crit Rev Biochem Mol Biol 34, 273-284.
Chitwood, D.J., Lusby, W.R., Lozano, R., Thompson, M.J., and Svoboda, J.A. (1983). Novel nuclear methylation of sterols by the nematode Caenorhabditis elegans. Steroids 42, 311-319.
Entchev, E.V., and Kurzchalia, T.V. (2005). Requirement of sterols in the life cycle of the nematode Caenorhabditis elegans. Semin Cell Dev Biol 16, 175-182.
Folch, J., Lees, M., and Sloane Stanley, G.H. (1957). A simple method for the isolation and purification of total lipides from animal tissues. J Biol Chem 226, 497-509.
Gaber, R.F., Copple, D.M., Kennedy, B.K., Vidal, M., and Bard, M. (1989). The yeast gene ERG6 is required for normal membrane function but is not essential for biosynthesis of the cell-cycle-sparking sterol. Mol Cell Biol 9, 3447-3456.
Gaylor, J.L. (2002). Membrane-bound enzymes of cholesterol synthesis from lanosterol. Biochem Biophys Res Commun 292, 1139-1146.
Gems, D., Sutton, A.J., Sundermeyer, M.L., Albert, P.S., King, K.V., Edgley, M.L., Larsen, P.L., and Riddle, D.L. (1998). Two pleiotropic classes of daf-2 mutation affect larval arrest, adult behavior, reproduction and longevity in Caenorhabditis elegans. Genetics 150, 129-155.
Gerisch, B., and Antebi, A. (2004). Hormonal signals produced by DAF-9/cytochrome P450 regulate C. elegans dauer diapause in response to environmental cues. Development 131, 1765-1776.
Gerisch, B., Rottiers, V., Li, D., Motola, D.L., Cummins, C.L., Lehrach, H., Mangelsdorf, D.J., and Antebi, A. (2007). A bile acid-like steroid modulates Caenorhabditis elegans lifespan through nuclear receptor signaling. Proc Natl Acad Sci U S A 104, 5014-5019.
Gerisch, B., Weitzel, C., Kober-Eisermann, C., Rottiers, V., and Antebi, A. (2001). A hormonal signaling pathway influencing C. elegans metabolism, reproductive development, and life span. Dev Cell 1, 841-851.
Gietz, R.D., and Sugino, A. (1988). New yeast-Escherichia coli shuttle vectors constructed with in vitro mutagenized yeast genes lacking six-base pair restriction sites. Gene 74, 527-534.
Gill, M.S., Held, J.M., Fisher, A.L., Gibson, B.W., and Lithgow, G.J. (2004). Lipophilic regulator of a developmental switch in Caenorhabditis elegans. Aging Cell 3, 413-421.
Golden, T.R., and Melov, S. (2007). Gene expression changes associated with aging in C. elegans. WormBook, 1-12.
Grebenok, R.J., Galbraith, D.W., and Penna, D.D. (1997). Characterization of Zea mays endosperm C-24 sterol methyltransferase: one of two types of sterol methyltransferase in higher plants. Plant Mol Biol 34, 891-896.
Hughes, A.L., Lee, C.Y., Bien, C.M., and Espenshade, P.J. (2007). 4-Methyl sterols regulate fission yeast SREBP-Scap under low oxygen and cell stress. J Biol Chem 282, 24388-24396.
Jeong, P.Y., Jung, M., Yim, Y.H., Kim, H., Park, M., Hong, E., Lee, W., Kim, Y.H., Kim, K., and Paik, Y.K. (2005). Chemical structure and biological activity of the Caenorhabditis elegans dauer-inducing pheromone. Nature 433, 541-545.
Jia, K., Albert, P.S., and Riddle, D.L. (2002). DAF-9, a cytochrome P450 regulating C. elegans larval development and adult longevity. Development 129, 221-231.
Kagan, R.M., and Clarke, S. (1994). Widespread occurrence of three sequence motifs in diverse S-adenosylmethionine-dependent methyltransferases suggests a common structure for these enzymes. Arch Biochem Biophys 310, 417-427.
Kurzchalia, T.V., and Ward, S. (2003). Why do worms need cholesterol? Nat Cell Biol 5, 684-688.
Ludewig, A.H., Kober-Eisermann, C., Weitzel, C., Bethke, A., Neubert, K., Gerisch, B., Hutter, H., and Antebi, A. (2004). A novel nuclear receptor/coregulator complex controls C. elegans lipid metabolism, larval development, and aging. Genes Dev 18, 2120-2133.
Magaraci, F., Jimenez, C.J., Rodrigues, C., Rodrigues, J.C., Braga, M.V., Yardley, V., de Luca-Fradley, K., Croft, S.L., de Souza, W., Ruiz-Perez, L.M., et al. (2003). Azasterols as inhibitors of sterol 24-methyltransferase in Leishmania species and Trypanosoma cruzi. J Med Chem 46, 4714-4727.
Mak, H.Y., and Ruvkun, G. (2004). Intercellular signalling of reproductive development by the C. elegans DAF-9 cytochrome p450. Development 131, 1777-1786.
Martin, R., Dabritz, F., Entchev, E.V., Kurzchalia, T.V., and Knolker, H.J. (2008). Stereoselective synthesis of the hormonally active (25S)-delta7-dafachronic acid, (25S)-Delta4-dafachronic acid, (25S)-dafachronic acid, and (25S)-cholestenoic acid. Org Biomol Chem 6, 4293-4295.
Martin, R., Schmidt, A.W., Theumer, G., Krause, T., Entchev, E.V., Kurzchalia, T.V., Knölker, H-J. (2009). Synthesis and biological activity of the (25R-)cholesten-26-oic acids - ligands for the hormonal receptor DAF-12 in Caenorhabditis elegans. Org Biochem 7, 909-920
Martin, R., Entchev, E.V., Däbritz, F., Kurzchalia, T.V., and Knölker, H.-J. (2009). Synthesis and hormonal activity of the (25S-)cholesten-26-oic acids - ligands for the hormonal receptor DAF-12 in Caenorhabditis elegans. Eur J Org Chem in press.
Martin, R., Saini, R., Bauer, I., Gruner, M., Kataeva, O., Zagoriy, V., Entchev, E.V., Kurzchalia, T.V., and Knölker, H.-J. (2009). 4a-Bromo-5a-cholestan-3b-ol and nor-5a-cholestan-3b-ol derivatives-stereoselective synthesis and hormonal activity in Caenorhabditis elegans. Org Biomol Chem 7, 2303-2309.
Matyash, V., Entchev, E.V., Mende, F., Wilsch-Brauninger, M., Thiele, C., Schmidt, A.W., Knolker, H.J., Ward, S., and Kurzchalia, T.V. (2004). Sterol-derived hormone(s) controls entry into diapause in Caenorhabditis elegans by consecutive activation of DAF-12 and DAF-16. PLoS Biol 2, e280.
Matyash, V., Geier, C., Henske, A., Mukherjee, S., Hirsh, D., Thiele, C., Grant, B., Maxfield, F.R., and Kurzchalia, T.V. (2001). Distribution and transport of cholesterol in Caenorhabditis elegans. Mol Biol Cell 12, 1725-1736.
Motola, D.L., Cummins, C.L., Rottiers, V., Sharma, K.K., Li, T., Li, Y., Suino-Powell, K., Xu, H.E., Auchus, R.J., Antebi, A., et al. (2006). Identification of ligands for DAF-12 that govern dauer formation and reproduction in C. elegans. Cell 124, 1209-1223.
Nadler, S.A., De Ley, P., Mundo-Ocampo, M., Smythe, A.B., Patricia Stock, S., Bumbarger, D., Adams, B.J., De Ley, I.T., Holovachov, O., and Baldwin, J.G. (2006). Phylogeny of Cephalobina (Nematoda): molecular evidence for recurrent evolution of probolae and incongruence with traditional classifications. Mol Phylogenet Evol 40, 696-711.
Nes, W.D., Jayasimha, P., Zhou, W., Kanagasabai, R., Jin, C., Jaradat, T.T., Shaw, R.W., and Bujnicki, J.M. (2004). Sterol methyltransferase: functional analysis of highly conserved residues by site-directed mutagenesis. Biochemistry 43, 569-576.
Ogawa, A., Streit, A., Antebi, A., and Sommer, R.J. (2009). A conserved endocrine mechanism controls the formation of dauer and infective larvae in nematodes. Curr Biol 19, 67-71.
Patel, D.S., Fang, L.L., Svy, D.K., Ruvkun, G., and Li, W. (2008). Genetic identification of HSD-1, a conserved steroidogenic enzyme that directs larval development in Caenorhabditis elegans. Development.
Riddle, D.L., and Albert, P.S. (1997). Genetic and environmental regulation of dauer larva development. In Celegans II, D.L. Riddle, T. Blumenthal, J.B. Meyer, and J.R. Priess, eds. (Cold Spring Harbor Laboratory Press), pp. 739-768.
Rottiers, V., Motola, D.L., Gerisch, B., Cummins, C.L., Nishiwaki, K., Mangelsdorf, D.J., and Antebi, A. (2006). Hormonal control of C. elegans dauer formation and life span by a Rieske-like oxygenase. Dev Cell 10, 473-482.
Smith, H.L. (2000). Investigating development of infective stage larvae of filarial nematodes. Front Biosci 5, E95-E102.
Swanson, M.M., and Riddle, D.L. (1981). Critical periods in the development of the Caenorhabditis elegans dauer larva. Dev Biol 84, 27-40.
Viney, M.E. (2009). How did parasitic worms evolve? Bioessays.
Visbal, G., Marchan, E., Maldonado, A., Simoni, Z., and Navarro, M. (2008). Synthesis and characterization of platinum-sterol hydrazone complexes with biological activity against Leishmania (L.) mexicana. J Inorg Biochem 102, 547-554.
Wilm, T., Demel, P., Koop, H.U., Schnabel, H., and Schnabel, R. (1999). Ballistic transformation of Caenorhabditis elegans. Gene 229, 31-35.
Zhou, W., Lepesheva, G.I., Waterman, M.R., and Nes, W.D. (2006). Mechanistic analysis of a multiple product sterol methyltransferase implicated in ergosterol biosynthesis in Trypanosoma brucei. J Biol Chem 281, 6290-6296.

## Claims

1. A compound for use as an anti-helminthic agent, said compound being selected from
(i) a compound interfering with the transcription or translation of a nucleic acid sequence encoding a polypeptide having a function of introducing a methyl group at a specific position into the sterol nucleus of a sterol which nucleic acid sequence is
(a) a nucleic acid sequence encoding a polypeptide comprising or consisting of the amino acid sequence of SEQ ID NO: 1;
(b) a nucleic acid sequence comprising or consisting of the sequence of SEQ ID NO: 2;
(c) a nucleic acid sequence which encodes a polypeptide which is at least 70% identical to the sequence of SEQ ID NO: 1;
(d) the nucleic acid sequence of (b) wherein T is U;
(e) a nucleic acid sequence hybridizing to the complementary strand of a nucleic acid molecule of any of (a) to (d); or
(f) a nucleic acid sequence being degenerate to the sequence of the nucleic acid molecule of (d) or (e),
wherein said compound is an antisense molecule, siRNA, shRNA, miRNA, aptamer, antibody or a ribozyme, and
(ii) a compound interfering with the function of the polypeptide encoded by the nucleic acid sequence of (i), said compound being an antibody, enzyme, peptide or a small organic molecule.

2. The compound of claim 1, wherein the polypeptide has a function of introducing a methyl group at a specific position into the A-ring of the sterol nucleus of a sterol.

3. The compound of claim 2, wherein the polypeptide has a function of introducing a methyl group into position C-4 of the A-ring of a sterol.

4. The compound of any of claims 1 to 3, wherein the sterol is a cholesterone, cholesterol, ergosterol, ergosterone, sitosterol or sitosterone.

5. The compound of claim 4, wherein the cholesterone is dafachronic acid.

6. The compound of claim 5, wherein the dafachronic acid is Δ⁴-dafachronic acid, Δ⁷-dafachronic acid, Δ¹⁸⁽¹⁴⁾-dafachronic acid or saturated dafachronic acid.

7. The compound of any one of claims 1 to 6, wherein the small organic molecule is selected from the formula of a), b), c), d), or e):
a) 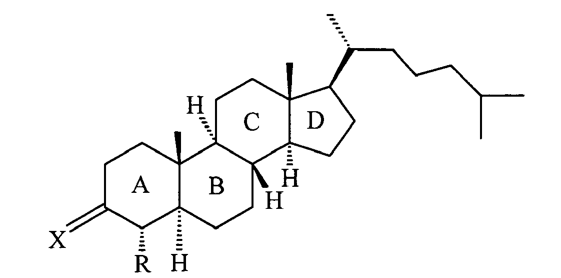 X = O, S, NOH;
R = H, C≡CH, C≡N, N=N⁺X⁻, N₃, COOH, NO₂, Cl, Br, I;
A,B,C,D = are rings in which at least one bond can be unsaturated
b) 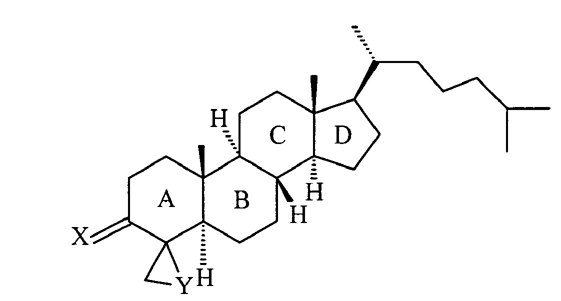 X = O, S, NOH;
Y = O, S, NR (R = alkyl, benzyl, aryl);
A,B,C,D = are rings in which at least one bond can be unsaturated
c) 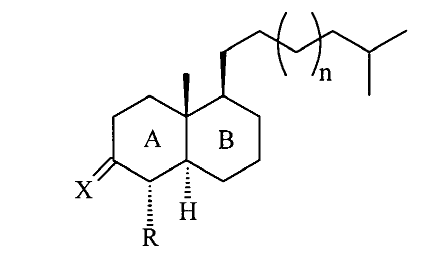 n = 0 - 9;
X = O, S, NOH;
R = H, C≡CH, C=N, N=N⁺X⁻, N₃, COOH, NO₂, Cl, Br, I;
A,B = are rings in which at least one bond can be unsaturated
d) 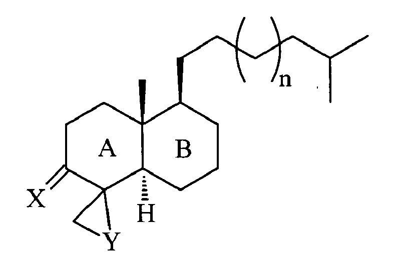 n = 0 - 9;
X = O, S, NOH;
Y = O, S, NR (R = alkyl, benzyl, aryl);
A,B = are rings in which at least one bond can be unsaturated
e) 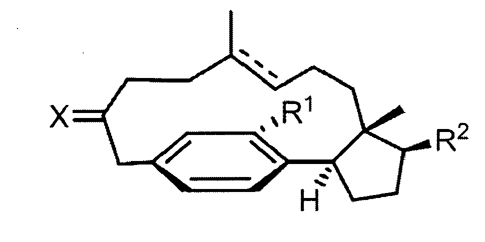 X = O, S, NOH
R¹ = CHO, CH₃, CH₂OH, COOH, COOMe, COMe R² = CH(CH₃)CH₂CH₂CH₂CH(CH₃)₂,
CH(CH₃)CH=CHCH(CH₃)CH(CH₃)₂

8. The compound of any one of claims 1 to 7, wherein the anti-helminthic agent is an anti-nematode agent.

9. A plant protective agent comprising the compound as defined in any of claims 1 to 8.

10. A pharmaceutical composition comprising the compound as defined in any of claims 1 to 8.

11. A transgenic multi-cellular organism carrying
(a) the nucleic acid sequence as defined in claim 1; or
(b) a vector comprising the nucleic acid sequence as defined in claim 1 episomally or in the germ line.
